# EUROPEAN PATENT SPECIFICATION

(11) **EP 4 114 322 B1**
(45) Date of publication and mention of the grant of the patent: **31.07.2024**
(21) Application number: 21764767.6
(22) Date of filing: 01.03.2021
(51) Int. Cl.: A61F 2/66, A61F 2/00, A61F 2/50, A61F 2/60, A61F 2/62, A61F 2/76

(54) **ADAPTER FOR PROSTHETIC FOOT**
ADAPTER FÜR FUSSPROTHESE
ADAPTATEUR POUR PIED PROTHÉTIQUE

(30) Priority: 02.03.2020 US 202062984170 P
(43) Date of publication of application: 11.01.2023
(73) Proprietor: Otto Bock HealthCare LP, Austin, TX 78758 (US)
(72) Inventor: ANDERSON, Vaughn Roy, Highland, Utah 84003 (US); DAY, Jesse, Holladay, Utah 84117 (US); MORAMPUDI, Vijay, South Jordan, Utah 84009 (US); RUSH, Douglas E., Salt Lake City, Utah 84109 (US)
(74) Representative: Gramm, Lins & Partner Patent- und Rechtsanwälte PartGmbB
(86) International application number: PCT/US2021/020345
(87) International publication number: WO 2021/178333

(56) References cited:
- WO-A2-2005/112838
- WO-A2-2006/034285
- WO-A2-2006/034285
- US-A- 3 480 972
- US-A- 5 482 513
- US-A1- 2002 040 249
- US-A1- 2003 120 354
- US-A1- 2007 027 557
- US-A1- 2013 123 942
- US-B2- 8 118 879

## Description

The present disclosure relates to a prosthetic foot comprising an adapter and an elongate support member of carbon filament material having a first surface. The adapter comprises a compressible member, a base portion, a cavity sized to receive the compressible member and a connector portion extending from the base portion and configured to releasably secure the prosthetic foot to a prosthesis.

Many prosthetic feet of different designs are directed at improving stability, roll-over, and gait characteristics of the prosthetic foot. One approach has been to incorporate springs to store and release energy during motion of the prosthetic foot. Such springs can be of different shapes, such as C-shaped or U-shaped. However, such foot designs tend to be bulky and arguably do not improve the foot rollover.

Another approach is to provide a foot member that is split along at least a portion of its length. The split foot member is capable of flexing substantially independently of each other. This provides a prosthetic foot with increased stability. However, a split-toe foot alone may not provide the desired fluid range of motion of a human foot's natural stride.

Other designs incorporate a bearing or ball joint to provide one or more axes of rotation to mimic the human ankle joint. Elastomeric bumpers are commonly used to control rotation about the axes and because the bumpers are not constrained from expanding in a lateral direction relative to the applied force, these elastomers suffer from creep and compression set resulting in declining performance over time and periodic replacement. Bearings and ball joints also undergo wear that results in mechanism play and noise, and potentially added cost and weight for the device.

Yet other designs attempt to provide a prosthetic foot capable of a smooth roll-over by incorporating passive hydraulic systems, which create damping and smooth roll-over. However, this creates a foot that is more complex, requires additional maintenance, is heavier and more expensive, and the hydraulic nature of the foot counters the principle of energy return.

Still other designs attempt to provide a prosthetic foot capable of a smooth roll-over by defining the gap beneath the pyramid adapter. Accordingly, the gap beneath the pyramid, in these designs, which is defined by a constant angle between on the adapter base and the carbon spring, attempt to improve roll-over. However, during the gait cycle, as soon as this constant-angle gap is closed, the foot spring becomes instantly stiff (a stepped stiffness characteristic), which in turn interrupts a "smooth" roll-over of the foot. This approach also creates a stress concentration in the upper foot plate (also referred to as a top spring member) at the end of the adapter.

The connection between the prosthetic adapter and the upper foot plate is often a challenge for prosthetic foot designers. This is typically the location which experiences the most extreme stress in a prosthetic foot, therefore is important to optimize the connection to maximize both foot durability and flexibility. Over time, prosthetic foot designers have improved the connection between prosthetic adapters and the remaining components of a foot. Prosthetic adapters are typically made of metal and the upper foot plate is typically made of a fiber reinforced composite lam inate.

A first generation of a prosthetic adapter interface was flat and square or rectangular with four bolt holes at the corners of the interface: two at the anterior end and two at the posterior end of the adapter. This resulted in high stresses in the anterior bolts, high contact stresses in the upper foot plate at the anterior edge of the pyramid, and poor roll-over characteristics for the foot.

WO 2006/034285 A3 relates to a prosthetic foot comprising an adapter and an elongate support member of carbon filament material having a first surface. The adapter comprises a compressible member, a base portion, a cavity sized to receive the compressible member and a connector portion extending from the base portion and configured to releasably secure the prosthetic foot to a prosthesis.

US 8 118 879 B2 relates to a keel for a prosthetic foot with a comprises a unitary keel body having a longitudinal axis and a length. The keel body includes a forefoot portion, a heel portion, and an ankle portion extending therebetween. In addition, the keel comprises a first bumper disposed in a first capture cavity in the ankle portion.

The first bumper has a central axis that is substantially perpendicular to the longitudinal axis of the keel body in top view.

US 5 482 513 A relates to a lightweight foot prosthesis, comprising: a foot, an ankle joint affixed to said foot and capable of rotation about a transverse axis. The joint comprises a body having a transverse bore therethrough and a shell. The shell includes an integral axle extending through the bore such that the shell is pivotable with respect to the body. A snubber is arranged at an anterior portion of the shell to dampen a contact between the shell and the foot. The foot prosthesis comprises, thus, an elongate support member having a top surface and an adapter comprising a compressible member (snubber), a base portion, a cavity sized to receive the compressible member, a connector portion extending from the base portion and configured to releasably secure the prosthetic foot to a prosthesis, wherein the base portion comprises a contact surface arranged to contact the top surface of the elongate support member. Application of a force to the connector portion compresses the compressible member to dampen movement of the base portion relative to the support member.

For these and other reasons, there is a need to provide improved adapters for prosthetic devices, particularly for prosthetic feet.

In order to satisfy numerous patients and their specific circumstances, a foot spring with a smoothly increasing stiffness may be advantageous for the foot and its performance. One aspect of the present disclosure relates to a prosthetic foot having a relatively soft spring property for slow walking and a relatively stiff spring property for faster walking and more aggressive activities. One objective of the present disclosure is to reduce or eliminate an abrupt transition in stiffness, such as those prevalent in overload spring designs and in designs that utilize a constant-angle on the base of the pyramid adapter. The large radius or elliptical shape of the base of the pyramid adapter in at least some embodiments disclosed herein results is a smoother roll-over characteristic of the foot over a wide variety of user activities. Another aspect of the present disclosure relates to an adapter for a prosthetic foot. The adapter is fastened to the upper foot plate at a rearward or posterior location on the adapter. The distal or anterior end of the adapter forms a gap between the bottom surface of the adapter and the upper foot plate. The bottom distal surface of the adapter has a cavity that accepts a compressible member, such as a polymer element. During the gait cycle, the adapter rotates with respect to the upper foot plate and, as this rotation occurs, the gap between the adapter and the upper foot plate is reduced or eliminated. This reduction in gap results in the polymer material being compressed into the cavity on the bottom surface of the pyramid adapter. As the foot rolls over from heel strike to toe off, the upper foot plate rolls up against the anterior distal edge of the adapter, thus gradually compressing the polymer.

The initial overall stiffness of the prosthetic foot is controlled by the durometer of the polymer material in conjunction with the volume of the receiving cavity. As the polymer material is compressed into the cavity, it begins to fill the cavity. As the volume of the cavity is filled with the polymer, the polymer becomes stiffer. Once the cavity volume is filled completely with the polymer material, the polymer material becomes infinitely stiff, thus increasing the overall stiffness of the prosthetic foot. The increase in stiffness can be controlled by how much additional space is remaining in the cavity after the adapter is in full contact with the upper foot plate. If the polymer has a larger volume than the cavity, the upper foot plate will not contact the bottom surface of the adapter. The secondary stiffness of the prosthetic foot is present when the polymer either completely fills the cavity or the bottom surface of the adapter is in full contact with the upper foot plate. This secondary stiffness is determined solely by the spring elements (i.e., the upper foot plate).

As a result of these variables, the prosthetic foot can be configured to have an initial soft stiffness which increases to a high stiffness. Adjustments to stiffness can be made by replacing the polymer material with a higher or lower durometer polymer material. To relieve stresses at the connection point between the adapter and the foot spring members, the anterior mounting holes in the adapter may be moved further posterior and/or removed completely, and the radius on the anterior edge of the adapter may be increased. Some adapter designs have no anterior mounting holes and the radius at the distal anterior edge of the adapter is increased to allow the upper foot plate to roll up onto the anterior distal radius of the adapter. The contact surface of the adapter is typically planar from the posterior end through the location of the posterior mounting fasteners and then transitions to one or more angled surface portions and/or radius portions at the anterior end. As the upper foot plate bends upward and contacts the anterior edge of the adapter during roll over, the lever arm acting on the foot plate decreased and the contact point moves further forward. This decreases the bending stresses in the upper foot pate and stiffens the upper foot plate.

Another aspect of the present disclosure relates to a prosthetic foot having an adapter and a gap between a flexible upper foot plate and the bottom surface of the adapter. As the foot rolls over from heel strike to toe off, the upper foot plate rolls up against the anterior distal edge of the adapter. The gap may be defined, at least in part, by multiple radii or a continuously variable radius on the distal contact surface of the adapter.

In one example, a prosthetic foot includes an elongate support member of carbon filament material having a first surface, and an adapter. The adapter includes a compressible member, a base portion having a contact surface arranged to contact the first surface of the support member, a cavity sized to receive the compressible member, and a connector portion extending from the base portion and configured to releasably secure the prosthetic foot to a prosthesis, wherein application of a force to the connector portion compresses the compressible member to dampen movement of the base portion relative to the support member until the contact surface contacts the first surface.

The base portion may have a first end and a second end, the first end being fixed relative to the support member, and the second end being movable relative to the support member. The compressible member may be deformable to fill the cavity when compressed. The contact surface may have a contoured shape. The compressible member, when compressed, may be retained entirely within the cavity. The compressible member may include a polymeric material. The cavity may have a hemispherical shape. The contact surface may be spaced away from the first surface at the second end when the prosthetic foot is in a rest state prior to application of the force.

Another example relates to a prosthetic foot that includes a foot plate spring member, a top spring member secured to the foot plate spring member and having a first surface, and an adapter. The adapter includes a damping member, and a base portion having a first end, a second end, a cavity, and a contact surface arranged to contact the first surface of the top spring member, the first end being fixed relative to the top spring member, the second end being movable relative to the top spring member, and at least a portion of the damping member being positioned in the cavity. The adapter also includes a connector portion extending from the base portion and configured to releasably secure the prosthetic foot to a prosthesis, wherein application of a force to the connector portion the first surface of the base portion moves towards the first surface of the top spring member to compress the damping member until the contact surface contacts the first surface.

The damping member may include a polymeric, compressible material. The cavity may have a spherical or partial spherical shape. The damping member may dampen movement between the first end and the contact surface during application of the force. The top spring member may be connected to the foot plate spring member at an anterior end of the top spring member, the connector portion may be arranged vertically above the base portion, and the first end of the base portion may be positioned posterior of the second end.

A further example relates to a method of damping movement in a prosthetic device. The method includes providing a prosthetic device that includes an elongate composite spring member having a first surface, and an adapter having a base portion, the base portion having a contact surface arranged facing the first surface of the support member, a damping member interposed between the first surface and the base portion, and a connector portion extending from the base portion and configured to releasably secure the prosthetic device to a prosthesis. The method further includes applying a force to the connector portion to bend the elongate composite spring member and actuate the damping member to dampen movement of the base portion relative to the spring member until the contact surface contacts the first surface.

Actuating the damping member may include compressing the damping member. The base portion may include a cavity, and actuating the damping member may include deforming the damping member to increase a surface area of the damping member in contact with the cavity. The contact surface may have a first end and a second end, the first end being fixed relative to the first surface, and the second end being movable relative to the first surface in response to applying the force to the connector portion. The prosthetic device may be a prosthetic foot, the connector portion may be arranged vertically above the base portion, and the first end of the adapter may be positioned posterior of the second end.

Another example relates to a prosthetic foot that includes a flexible fiber reinforced spring member having a proximal surface, a shape of the proximal surface defined by a straight line in at least one direction, and a rigid prosthetic pyramid adapter having a base portion and a contact surface arranged to contact the spring member. The adapter is fixedly attached to the spring member with the contact surface facing the proximal surface of the spring member, and the contact surface is arranged parallel to the proximal surface over a portion of the proximal surface. A gap is formed between the contact surface of the adapter and the proximal surface of the spring member.

The shape of the gap may be formed by at least two radii surfaces, each of the at least two radii surfaces having a radius equal to or greater than about 0.64 cm (0.25 inch), and particularly equal to or greater than about 2.54 cm (1 inch), and being oriented convex with respect to the gap, and the spring member may be configured to flex to create pressure on both of the at least two radii surfaces during a gait cycle. The shape of a distal surface of the adapter may be formed by at least two radii surfaces, each of the at least two radii surfaces may have a radius equal to or greater than about 0.64 cm (0.25 inch), and particularly equal to or greater than about 2.54 cm (1 inch), and convex with respect to the gap, and the spring member may flex to create pressure on both of the at least two radii surfaces during a gait cycle. The prosthetic foot may be free of material interposed between the spring member and the adapter. The prosthetic foot may be free of material interposed between the spring member and the adapter.

A further example relates to an adapter for a prosthetic foot, the prosthetic foot including a top spring having a top surface. The adapter includes a distal portion having a contact surface arranged facing the top surface of the top spring, the contact surface having a recess formed therein, a proximal portion having a connection feature to connect the prosthetic foot to a lower leg prosthetic component, a posterior portion configured to connect the adapter to the top spring, and a resilient member positioned in the recess and arranged to contact the top surface of the top spring to provide damping between the adapter and the top spring during operation of the prosthetic foot.

The recess may have a hemispherical shape and the resilient member may include a hemispherical portion. The resilient member may include an elastomer material. The resilient member may include a planar surface arranged facing the top surface of the top spring. The contact surface may include an anterior end and a posterior end, the recess being spaced away from the anterior end. The recess may be positioned centrally on the adapter in a medial/lateral direction. The connection feature may include a pyramid connector. The resilient member may include a contoured surface that mates with a contoured surface of the recess.

Another example relates to a prosthetic foot with a top spring having a top surface, and an adapter. The adapter includes a distal portion having a contact surface arranged facing the top surface of the top spring, the contact surface having a variable radius of curvature including at least two radii equal to or greater than about 0.64 cm (0.25 inch), and particularly equal to or greater than about 2.54 cm (1 inch). The adapter also includes a proximal portion having a connection feature to connect the prosthetic foot to a lower leg prosthetic component, a posterior portion configured to connect the adapter to top spring, and a resilient member positioned arranged between the adapter and the top surface of the top spring to provide damping between the adapter and the top spring during operation of the prosthetic foot.

The contact surface may include a recess and the resilient member may be retained in the recess. The resilient member may include a substantially planar surface arranged to contact the top surface of the top spring and a contoured surface arranged to contact the adapter. The variable radius of curvature may increase in a posterior direction.

A further example relates to a prosthetic foot that includes a flexible fiber reinforced spring member and a pyramid adapter. The spring member has a toe end, a medial side, a lateral side, and a contact surface, the contact surface having a shape defined by a straight line in a medial/lateral direction. The pyramid adapter has a first end and a second end, a pyramid protrusion, and a contact surface that faces the contact surface of the spring member. The adapter is fixedly attached to the spring member. In an unloaded state of the prosthetic foot, a gap exists between the adapter and the spring member, the gap being defined by a shape of the contact surface of the adapter and the shape of the contact surface of the spring member, and the gap having an elliptical shape in an anterior-posterior direction. In a terminal stance of a forward moving gait cycle during use of the prosthetic foot a size of, the gap is reduced.

The pyramid adapter may be rigid. The gap may extend to a location beneath the pyramid protrusion. The pyramid adapter may include metal. The pyramid adapter may be formed as a monolithic structure.

Another example relates to a prosthetic device that includes a support member having a first surface, and an adapter. The adapter includes compressible member, a base portion having a variable radius contact surface arranged to contact the first surface of the support member. The contact surface includes at least two radii equal to or greater than about 0.64 cm (0.25 inch), and a connector portion extending from the base portion and configured to releasably secure the prosthetic device to a prosthesis. Application of a force to the connector portion compresses the compressible member to dampen movement of the base portion relative to the support member until the contact surface contacts the first surface.

The base portion may have a first end and a second end, the first end being fixed relative to the support member, and the second end being movable relative to the support member. The base portion may include a cavity sized to receive the compressible member. The compressible member may be deformable to fill the cavity when compressed. A first portion of the contact surface may have a contoured shape and a second portion of the contact surface may have a planar shape. The contoured shape may be an elliptical shape. The compressible member may include a polymeric material. The contact surface may be spaced away from the first surface at the second end when the prosthetic device is in a rest state prior to application of the force. The prosthetic device may be a prosthetic foot, the support member may be an elongate spring member, and the connector portion may be arranged vertically above the base portion.

A further example relates to a prosthetic device that includes a support member having a first surface, and an adapter. The adapter includes a base portion having a first end, a second end, and a contact surface arranged to contact the first surface of the support member, the first end being fixed relative to the support member, the second end being movable relative to the support member, and the contact surface having a multi-radius portion. The adapter also includes a connector portion extending from the base portion and configured to releasably secure the prosthetic device to a prosthesis, wherein application of a force to the connector portion moves the first end toward the first surface of the base portion until the contact surface contacts the first surface.

The multi-radius portion may include an elliptical shape. The first surface may have a contoured shape. The prosthetic device may include a damping member interposed between the base portion and the first surface, and application of the force may dampen movement between the first end and the contact surface. The prosthetic device may be a prosthetic foot, the support member may be an elongate spring member, the connector portion may be arranged vertically above the base portion, and the first end of the base portion may be positioned posterior of the second end.

Another example relates to a method of damping movement in a prosthetic device. The method includes providing a prosthetic device having a support member with a first surface, and an adapter having a base portion having a contact surface arranged facing the first surface of the support member. A damping member is interposed between the first surface and the base portion, and a connector portion extends from the base portion and is configured to releasably secure the prosthetic device to a prosthesis. The method also includes applying a force to the connector portion to actuate the damping member to dampen movement of the base portion relative to the support member until the contact surface contacts the first surface.

Actuating the damping member may include compressing the damping member. The base portion may include a cavity, and actuating the damping member may include filling the cavity with the damping member. The contact surface may have a first end, a second end, and a contoured shape between the first and second ends, the first end being fixed relative to the first surface, and the second end being movable relative to the first surface in response to applying the force to the connector portion. Applying the force to the connector portion may deform the support member until the first surface has a contoured shape that matches a contoured shape of the contact surface. The prosthetic device may be a prosthetic foot, the support member may be an elongate spring member, the connector portion may be arranged vertically above the base portion, and the first end of the adapter may be positioned posterior of the second end.

The foregoing has outlined rather broadly the features and technical advantages of examples according to the disclosure in order that the detailed description that follows may be better understood. Additional features and advantages will be described hereinafter. The conception and specific examples disclosed may be readily utilized as a basis for modifying or designing other structures for carrying out the same purposes of the present disclosure. Features which are believed to be characteristic of the concepts disclosed herein, both as to their organization and method of operation, together with associated advantages will be better understood from the following description when considered in connection with the accompanying figures. Each of the figures is provided for the purpose of illustration and description only, and not as a definition of the limits of the claims.

A further understanding of the nature and advantages of the embodiments may be realized by reference to the following drawings. In the appended figures, similar components or features may have the same reference label.
FIG. 1 is a perspective view of an example prosthetic foot in accordance with the present disclosure.
FIG. 2 is a side view of a portion of the prosthetic foot shown in FIG. 1.
FIGS. 3A-D are side views of the prosthetic foot shown in FIG. 1 in different actuated positions.
FIG. 4 is a side view of another example prosthetic foot in accordance with the present disclosure.
FIG. 5 is a cross-sectional view of a portion of the prosthetic foot shown in FIG. 4.
FIG. 6 is a bottom perspective view of an adapter of the prosthetic foot shown in FIG. 4.
FIG. 7 is a graph showing stiffness characteristics of the prosthetic foot shown in FIG. 1.
FIG. 8 is a graph showing stiffness characteristics for the prosthetic foot shown in FIG. 1 using different damping members.
FIG. 9 is a graph showing stiffness characteristics of the prosthetic foot shown in FIG. 1 based on activity intensity.
FIG. 10 is a graph showing stiffness characteristics of the prosthetic foot shown in FIG. 1 for different activity intensities and gait cycle phase.
FIG. 11 is a flow diagram showing steps of an example method in accordance with the present disclosure.
FIG. 12A is a side view of another example prosthetic foot in accordance with the present disclosure.
FIG. 12B is a side view of the prosthetic foot shown in FIG. 12A in a flexed position. FIG. 13 is a side view of another example adapter in accordance with the present disclosure.
FIG. 14 is a side view of another example adapter in accordance with the present disclosure.
FIG. 15 is a side view of another example adapter in accordance with the present disclosure.
FIG. 16 is a side view of another example adapter in accordance with the present disclosure.
FIG. 17 is a side view of another example adapter in accordance with the present disclosure.
FIG. 18 is a side view of another example adapter in accordance with the present disclosure.
FIG. 19A is a side view of another example adapter in accordance with the present disclosure.
FIG. 19B is a side view of the deflection of a spring member when mounted to the adapter shown in FIG. 19A.
FIGS. 19C-E are force profiles for the adapter of FIG. 19A.
FIGS. 20A-B are graphs illustrating force profiles for the adapter of FIG. 13.
FIGS. 21A-C are graphs illustrating force profiles for the adapter of FIG. 14.
FIGS. 22A-C are graphs illustrating force profiles for the adapter of FIG. 16.
FIGS. 23A-C are graphs illustrating force profiles for the adapter of FIG. 15.
FIG. 24 is a flow diagram showing steps of an example method in accordance with the present disclosure.
FIG. 25 is a flow diagram showing steps of an example method in accordance with the present disclosure.

While the embodiments described herein are susceptible to various modifications and alternative forms, specific embodiments have been shown by way of example in the drawings and will be described in detail herein. However, the exemplary embodiments described herein are not intended to be limited to the particular forms disclosed. Rather, the instant disclosure covers all modifications, equivalents, and alternatives falling within the scope of the appended claims.

### Detailed Description

The present disclosure is generally directed to prosthetic devices, and more particularly relates to adaptors (e.g., pyramid adapters) for prosthetic devices, and related methods for using such adaptors.

In order to satisfy numerous patients and their specific circumstances, a foot spring with a smooth increasing stiffness is one aspect of the foot and its performance. The present disclosure provides a soft spring for slow walking and a stiffer spring for faster walking and more aggressive activities. The present disclosure reduces or eliminates an abrupt transition in stiffness (as is prevalent in overload spring designs and in designs that utilize a constant angle on the bottom surface of the adapter). The addition of a damping element, such as a compressible polymeric member, allows for a customized and/or adjustable configurations that may be accomplished by the practitioners and that can be optimized for the individual user, such as providing a smooth roll-over characteristic of the foot over a wide variety of user activities.

The adapter, and particularly the combination of the adapter with a prosthetic foot, provides a lower stiffness for the prosthetic foot earlier in the gait cycle (e.g., during mid-stance and initial roll over), which increases gradually into a firmer stiffness characteristic during the more demanding portions of the gait cycle (e.g., during completion of roll over and toe off).

Lower limb prosthetic components may benefit from angular adjustment and are typically connected using an industry standard prosthetic pyramid connection. A prosthetic pyramid connection consists of a male pyramid connector/adapter and a complementary female pyramid connector/adapter connected to each other. The combination of the male and female adapters may provide for angular adjustment between two prosthetic components. The male portion may include two primary features: a pyramid protrusion and a contoured (e.g., spherical) surface. The pyramid protrusion may have four planar surfaces that are oriented in posterior, anterior, medial, and lateral directions. These surfaces may be angled with respect to a pyramid axis, wherein the pyramid axis extends along a longitudinal axis of the shin or thigh of the limb. The pyramid surfaces typically are angled in the range of about 10 degrees to about 30 degrees, and more particularly about 15 degrees. Due to the angles of the four pyramid surfaces, the protrusion necks down in a distal direction. The necked down end transitions to the contoured (e.g., spherical) surface. The contoured portion may be part of a separate base component with the pyramid protrusion fixedly and rigidly attached to the base component. Alternatively, the base component may be integrated to the spherical feature where the two features are combined into a single monolithic block of material. The pyramid protrusion may be threaded into the base component with the threads glued or otherwise fixed to prevent unthreading. Alternate methods of fixedly attaching a pyramid protrusion to a base component including a spherical surface are possible such as, for example, creating a stud on the narrow end of the pyramid protrusion and molding the stud into a fiber reinforced moldable base material, or by deforming the stud such that the stud creates a strong interference fit between the a pyramid protrusion and the base component. A male pyramid adapter may be monolithic meaning it is formed or composed of a single, continuous material without joints or seams.

A female pyramid component may include a predominantly hollow cylinder with a spherical surface formed on one end and four threaded fasteners The inner surface of the cylinder may not be round or cylindrical as recesses are commonly formed on this surface to allow increased articulation of the male protrusion within the cylinder while adjusting the angle between the components. The spherical surfaces of both the male and female components have a near identical spherical radius to allow mating with each other. Fasteners (e.g., two or more fasteners) may be threaded into the cylinder at an angle relative to the cylinder axis (e.g., 15 degree angle), and the fasteners may engage one or more of the four planar surfaces of the male pyramid protrusion to releasably secure the position of the pyramid connection. By adjusting the depth of the fasteners in the female component, the angle between the male and female pyramid components can be changed and the angle between two prosthetic components can be adjusted. A female pyramid component may be referred to as a pyramid receiver. A female pyramid adapter may be monolithic. The threaded fasteners typically are separate components in a monolithic female pyramid adapter.

The male and female pyramid adapters may each have fastening provisions to be attached to adjacent components, such as holes for attaching the pyramid adapter to components of a prosthetic foot or prosthetic knee using, for example, fasteners (e.g., bolts or rivets), a clamp, or a bonding surface for bonding the adapter to an adjacent component such as prosthetic pylon, which may be, for example, a composite or metal tube. A male or female pyramid connection component may be machined or formed directly onto a prosthetic device, for example, a prosthetic knee. For the purposes of this disclosure, a pyramid adapter may be either the male or female component of a pyramid connection and include either a pyramid protrusion and a spherical mating surface in the case of a male pyramid adapter or a spherical mating surface with multiple (e.g., four) threaded fasteners to engage and lock a pyramid protrusion in the case of a female adapter. A pyramid adapter may be fabricated separately from other components and include design features allowing the adapter to be attached to other prosthetic components in addition to connecting the complementary opposite component of a pyramid connection.

The present disclosure relates to prosthetic feet that typically include a rigid pyramid adapter. A rigid pyramid adapter does not allow movement within the adapter. Some pyramid adapters include one or more axes of rotation within the adapter which allows the pyramid protrusion to rotate relative to the base of the adapter. Rotation about the axes may be controlled or limited by a hydraulic circuit, a bumper, or a mechanism that allows the heel height of the foot to be adjusted. The devices of the present disclosure are generally directed to pyramid adapters that are rigid and are intended to provide angular adjustment between the male and female adapter components of the pyramid connection, but not movement or articulation within either the male or female adapter components.. Pyramid adapters that provide movement and adjustment within the adapter are typically more expensive to manufacture and may suffer from wear at surfaces associated with the axes that allow movement and any other component that restricts, controls, or prevents movement. This may result in reduced reliability. Prosthetic feet with such adapters may be referred to as hydraulic ankles, hydraulic feet, single axis feet, or adjustable heel height feet. A consequence of the high loads pyramid connections are subjected to and the desire to minimize the weight of all prosthetic components, is that a rigid pyramid adapter may exhibit a small amount of elastic deformation under the high forces imposed during a walking gait cycle, thus resulting in a small amount of angular change (e.g., less than two degrees) between any two surfaces within a pyramid connection.

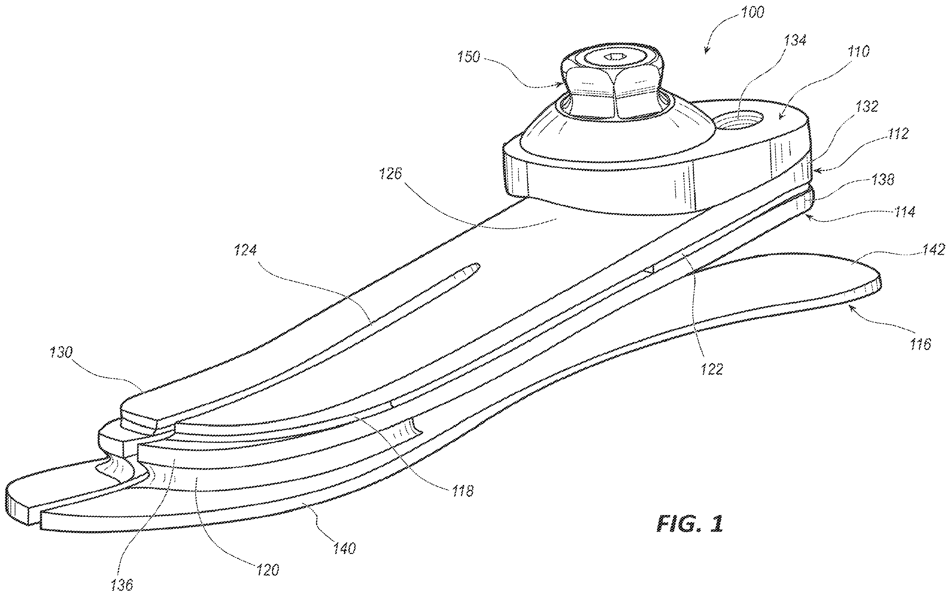

The damping member helps control the initial contact between the upper plate of the foot and the bottom surface of distal/anterior end of the adapter. As the upper plate rolls into contact with the adapter, the moment arm of the foot plate shortens, thereby stiffening the foot. Until the upper plate contacts the distal surface of the adapter, the stiffness is controlled by the damping member. The roll over characteristics of a prosthetic foot of the present disclosure can be adjusted by simply changing the damping member on the adapter. A stiffer damping member (e.g., a harder durometer polymer member) will stiffen the foot in its initial phase, whereas a less stiff damping member (e.g., a softer durometer polymer member) will soften the foot. Referring now to FIG. 1, an example prosthetic device 100 in the form of a prosthetic foot is shown and described. The prosthetic device 100 includes an adapter 110, a top spring member 112 (also referred to as a first spring member or an upper foot plate), a second spring member 114 (also referred to as a bottom spring member or bottom foot plate), and foot plate spring member 116 (also referred to as a sole spring or sole foot plate). The top and second spring members 112, 114 are secured together with a first bond connection 118. The second spring member 114 is secured to the foot plate spring member 116 with a second bond connection 120. A spacer 122 may be provided between the top and second spring members 112, 114. The prosthetic device 100 may include a medial/lateral slot 124 that extends from a toe end in a proximal or posterior direction along the length dimension of the prosthetic device 100. The adapter 110 is mounted to a top surface 128 of the top spring member 112 and is configured to releasably secure the prosthetic device 100 to another prosthetic component such as a prosthetic socket, pylon, or the like.

The top spring member 212 includes, in addition to the top surface 128, an anterior end 130, a posterior end 132, and one or more fastening bores 134. The second spring member 114 includes anterior and posterior ends 136, 138. The foot plate spring member 116 also includes anterior and posterior ends 140, 142. The first bond connection 118 secures the top and second spring members 112, 114 to each other at the anterior ends 130, 136. The spacer 122 is positioned between the top and second spring members 112, 114 at the posterior ends 132, 138. The second bond connection 120 may be used to secure the anterior end 136 of the second spring member 114 to the anterior end 140 of the foot plate spring member 116. In at least some arrangements, the anterior ends 130, 136 are spaced proximal of an anterior or distal-most end of the foot plate spring member 116.

Various arrangements are possible for the top, second and foot plate spring members 112, 114, 116 to provide desired characteristics for a particular prosthetic device. The arrangement shown in FIG. 1 provides the posterior end 142 of the foot plate spring member 116 freely moveable vertically relative to the top and second spring members 112, 114, while the anterior ends 130, 136, 140 of the top, second and foot plate spring members 112, 114, 116 are fixed relative to each other.

The medial/lateral slot 124 provides some relative movement between medial and lateral sides of the prosthetic device 100 at the anterior end. As noted above, the medial/lateral slot 124 may provide improved stability for the user, particularly when walking on uneven ground.

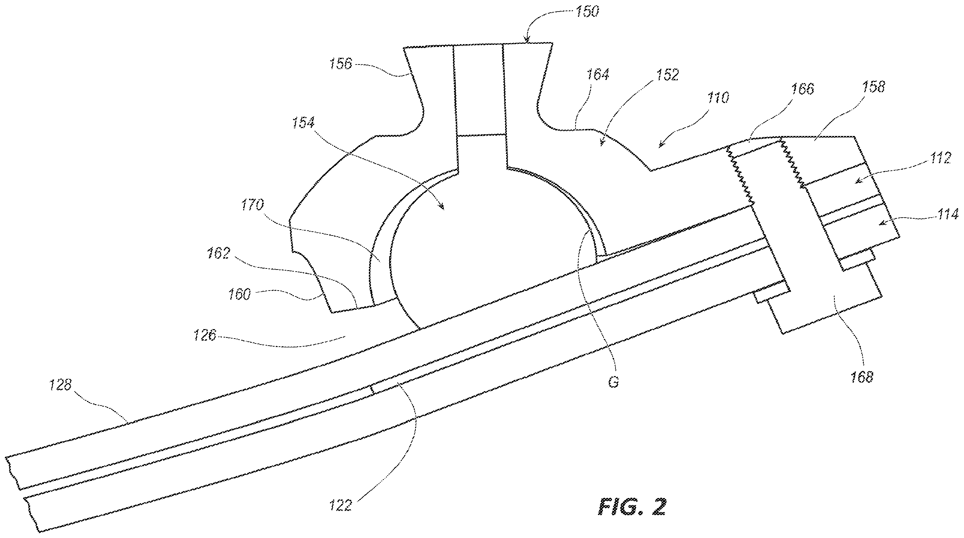

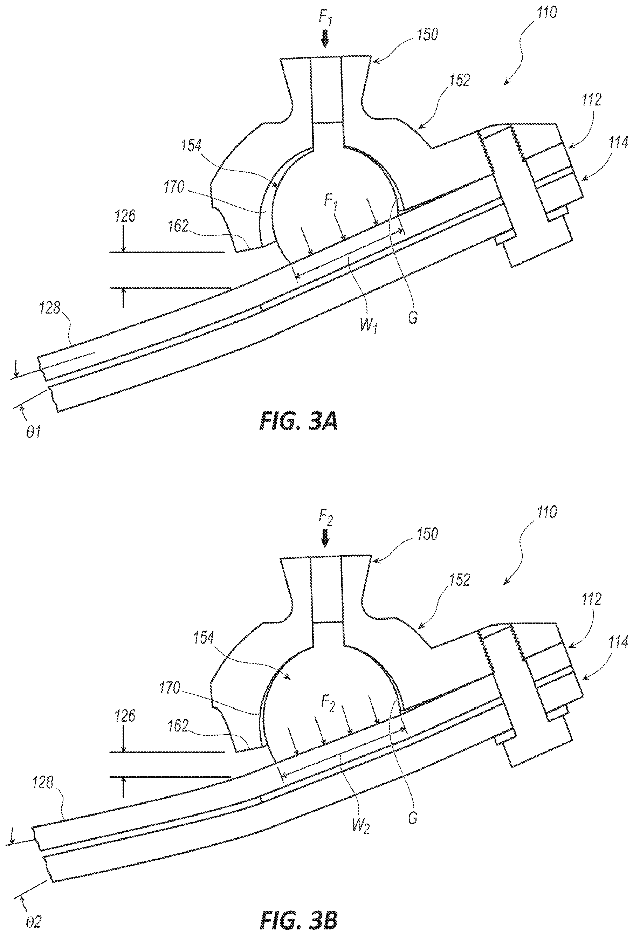

The adapter 110 may include a pyramid protrusion or connector 150, a base 152, and a damping member 154. The connector 150 includes a plurality of pyramid connector surfaces 156. The base 152 includes first and second ends 158, 160, a bottom contact surface 162, a top surface 164, a plurality of fastener apparatuses 166, and a cavity 170 sized to receive the damping member 154 (see FIGS. 2-3D). The connector 150 is mounted to the top surface 164 of the base. A portion of the contact surface 162 at the first end 158 is in contact with the top surface 128 of the top spring member 112. The fasteners 168 secure the first end 158 of the base 152 to the top spring member 112 to provide a fixed connection. The second end 160 of the base 152 is moveable vertically relative to the top surface 128 of the top spring member 112 during use of the prosthetic device 100. The description below related to FIGS. 3A-D relates to this relative movement between the second end 160 of the base 152 and the top spring member 112. Initially, prior to use of the prosthetic device 100 (e.g., application of a force at the connector 150), a space 126 is provided between the second end 160 and the top surface 128.

The cavity 170 may have a contoured shape such as a concave shape. The cavity 170 may have a hemispherical shape. The damping member 154 may also have a spherical or semi-spherical shape, or at least have a portion of a sphere or hemisphere as its shape. Many other shapes are possible for the cavity 170 and damping member 154 including, for example, cubical shape or a flat and thin shape. Prior to use, the damping member 154 may have a size that is smaller than the size of the cavity 170 thereby providing a gap G between the damping member 154 and surfaces of the cavity 170.

The damping member 154 may include a compressible material. In one example, the damping member 154 includes a polymeric material that is compressible and/or deformable upon application of a force and returns to the materials original shape after removal of the force. In at least some arrangements, the damping member includes an elastomeric material. Some example materials that are possible for the damping member 154 include the following types of rubbers or elastomers: polyisoprene, polybutadiene, polychloroprene, butyl, chloro butyl, bromo butyl, styrene-butadiene, nitrile, hydrogenated nitrile, ethylene propylene, ethylene propylene diene, epichlorohydrin, polyacrylic, polysulfide, silicone, thermoplastic and thermoset urethanes, fluorosilicone, fluoroelastomers, perfluoroelastomers, polyether block amides, polyester-polyether copolymers, polyamide-polyether copolymers, chlorosulfonated polyethylene, ethylene-vinyl acetate, thermoplastic elastomers, polyolefinic elastomers, and mixtures or block copolymers of the above.

Referring now to FIGS. 3A-D, the adapter 110 is shown in use with the prosthetic device 100 during operation (i.e., during use of the prosthetic device 100 in a gait cycle). In a first arrangement shown in FIG. 3A, an initial force F₁ is applied at the connector 150. The force F₁ is translated through the damping member 154 to the top surface 128 of the top spring member 112. In at least one example, the initial force F₁ is zero. The damping member 154 has an initial width W₁ wherein the damping member 154 interfaces with the top surface 128. Further, the space 126 between the second end 160 of the base 152 and the top surface 128 is at its maximum, and the gap G between the damping member 154 and surfaces of the cavity 170 is also at its maximum. The top spring member 112 is bent at an angle θ₁ along its length.

The force F₁ may represent the force applied prior to contacting the prosthetic foot on a ground surface such as during a swing phase of a gait cycle. In one example the force F₁ equals zero force, and may represent the prosthetic device in a rest state, in use during the swing phase of a gait cycle, or a state prior to use or a state in which the force applied is negligible.

FIG. 3B illustrates application of a greater force F₂ to the connector 150. The force F₂ is translated through the damping member 154 to the top spring member 112, which may compress and/or deform the damping member 154 to have a greater width W₂ and reduce the gap G within the cavity 170. The space 126 between the second end 160 and the top surface 128 may be reduced as compared to what is shown in FIG. 3A. Further, the top spring member 112 may flex or bend to a greater degree as shown by the increased angle θ₂. FIG. 3B illustrates that the damping member 154 is beginning to deform to fill the cavity 170. As the damping member 154 is compressed, it will ultimately conform to the geometry and shape of the cavity 170 while permitting the second end 160 of the base 152 to move closer to the top surface 128 of the top spring member 112.

The force F₂ may represent the force applied to the prosthetic foot upon heel strike during a gait cycle. The force F₂ may alternatively, or in addition, represent the force applied to the prosthetic foot during initial stages of roll-over during the stance phase of the gait cycle.

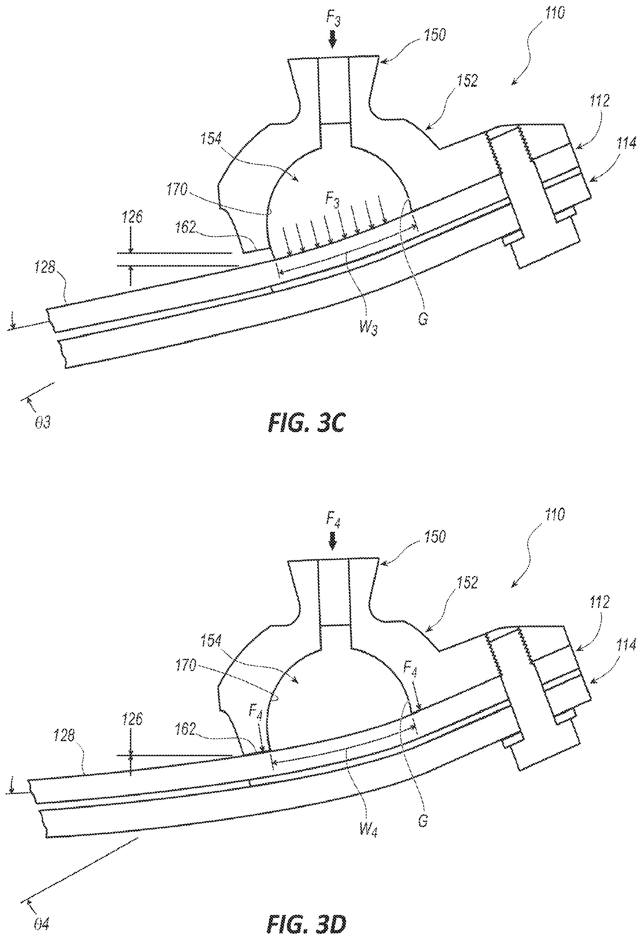

FIG. 3C illustrates application of a greater force F₃ to the connector 150, which is translated through the damping member 154 to the top spring member 112. Application of the force F₃ may further deform the damping member 154 such that the width is further widened to a width W₃, the gap G between the damping member 154 and the cavity 170 is further reduced, and the space 126 is also further reduced. FIG. 3C illustrates the damping member 154 nearly completely filling the cavity 170 while maintaining a space between the second end 160 and the top surface 128. The top spring member 112 is further bent or deformed to provide an increased angle θ₃ as the second end 160 moves closer to the top surface 128.

The force F₃ may represent application of a greater force, which is typically applied to the prosthetic foot during roll-over in the stance phase of a gait cycle. Generally, the force F₃ represents the prosthetic foot being significantly loaded by the weight and movement of the user. The damping member 154 shown in FIG. 3C has nearly completely filled the cavity 170, and the force transmitted from the connector 150 to the top spring member 112 has also increased significantly. Although the amount of force transmitted from the connector 150 to the top spring member 112 has increased as compared to what is shown in FIGS. 3A and 3B, it is a lesser amount of force as compared to when the base 152 is in contact with the top surface 128 along its entire length from the first end 158 to the second end 160.

The cavity (170) constrains the expansion of the damping member (154) in the directions perpendicular to the applied force (Poisson's expansion) and hence limits the compression set the damping member experiences, which increases the life of the damping member. How the damping member contacts the cavity affects the resulting stiffness of the damping member. For example if the damping member does not contact the cavity wall until the member is fully or almost fully compressed, the damping member is relatively soft until contact is made and then becomes very stiff. If the amount of surface area in contact between the cavity and the damping member increases linearly with compression of the damping member, the stiffness of the damping member increases more linearly. Controlling the rate at which the damping member contacts the cavity is one method of controlling the stiffness of the damping member as it is compressed.

FIG. 3D illustrates a greater force F₄ that is applied to the connector 150 and translated through the base 152 directly to the top surface 128 of the top spring member 112. The damping member 154 is completely compressed and contained entirely within the cavity 170, thereby providing limited force transmission because the force F₄ is primarily transferred through the stiffer structure of the base 152 to the top spring member 112. The resulting stiffness of the configuration shown in FIG. 3D is typically directly proportional to the stiffness of the top spring member 112 (in combination with the other spring members 114, 116) and the shape of the distal contact surface of the adapter, and at this point the stiffness is relatively unaffected by the damping member 154.

In FIG. 3D, there is no longer a gap G between the damping member 154 and the cavity 170. Further, the space 126 is reduced to zero. The width W₄ is at its maximum. The top spring member 112 is flexed to a greater angle θ₄ and has a shape along its top surface 128 that matches the shape of the contact surface 162. In at least some examples, the contact surface 162 has a contoured shape. The top surface 128 of the top spring member 112 may also have a contoured shape when in the maximum flexed arrangement shown in FIG. 3D.

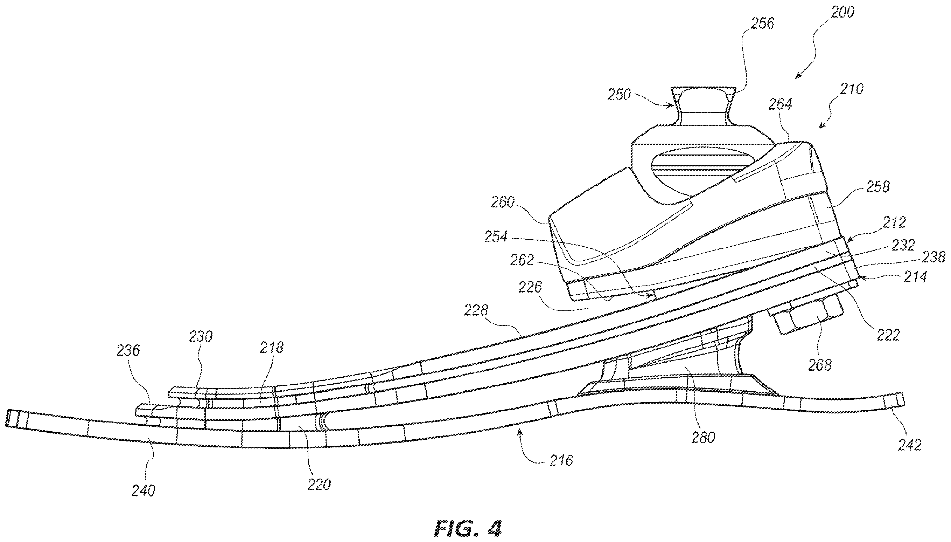

Referring now to FIG. 4, an example prosthetic device 200 in the form of a prosthetic foot is shown and described. The prosthetic device 200 includes an adapter 210, a top spring member 212, a second spring member 214, and foot plate spring member 216. The top and second spring members 212, 214 are secured together with a first bond connection 218. The second spring member 214 is secured to the foot plate spring member 216 with a second bond connection 220. A spacer 222 may be provided between the top and second spring members 212, 214. A heel damping member 280 is positioned between the second and foot plate spring members 214, 216 in the heel area of the prosthetic device 200. The adapter 210 is mounted to a top surface 228 of the top spring member 212 and is configured to releasably secure the prosthetic device 200 to another prosthetic component such as a prosthetic socket, pylon, or the like.

The top spring member 212 includes an anterior end 230, a posterior end 232, and one or more fastening bores that receive fasteners 268. The second spring member 214 includes anterior and posterior ends 236, 238. The foot plate spring member 216 also includes anterior and posterior ends 240, 242. The first bond connection 218 secures the top and second spring members 212, 214 to each other at the anterior ends 230, 236. The spacer 222 is positioned between the top and second spring members 212, 214 at the posterior ends 232, 238. The second bond connection 220 may be used to secure the anterior end 236 of the second spring member 214 to the anterior end 240 of the foot plate spring member 216. In at least some arrangements, the anterior ends 230, 236 are spaced proximal of an anterior or distal most end of the foot plate spring member 216. The arrangement shown in FIG. 4 provides the posterior end 242 of the foot plate spring member 216 freely moveable vertically relative to the top and second spring members 212, 214, while the anterior ends 230, 236, 240 of the top, second and foot plate spring members 212, 214, 216 are generally fixed relative to each other, although some slight relative movement (e.g., rotation, compression, translation) is possible depending on the type of materials used for the first and second bond connections 218, 220.

The adapter 210 may include a connector 250, a base 252, and a damping member 254. The connector 250 includes a plurality of pyramid connector surfaces 256. The base 252 includes first and second ends 258, 260, a bottom contact surface 262, a top surface 264, a plurality of fastener apparatuses 266, and a cavity 270 sized to receive the damping member 254. The connector 250 is mounted to the top surface 264 of the base. A portion of the contact surface 262 at the first end 258 is in contact with the top surface 228 of the top spring member 212. The fasteners 268 secure the first end 258 of the base 252 to the top spring member 212 to provide a fixed connection. The fasteners 268 are positioned posterior of the connector 250. The second end 260 of the base 252 is moveable vertically relative to the top surface 228 of the top spring member 212 during use of the prosthetic device 200. Initially, prior to use of the prosthetic device 200 (e.g., prior to application of a force at the connector 250), a space 226 is provided between the second end 260 and the top surface 228.

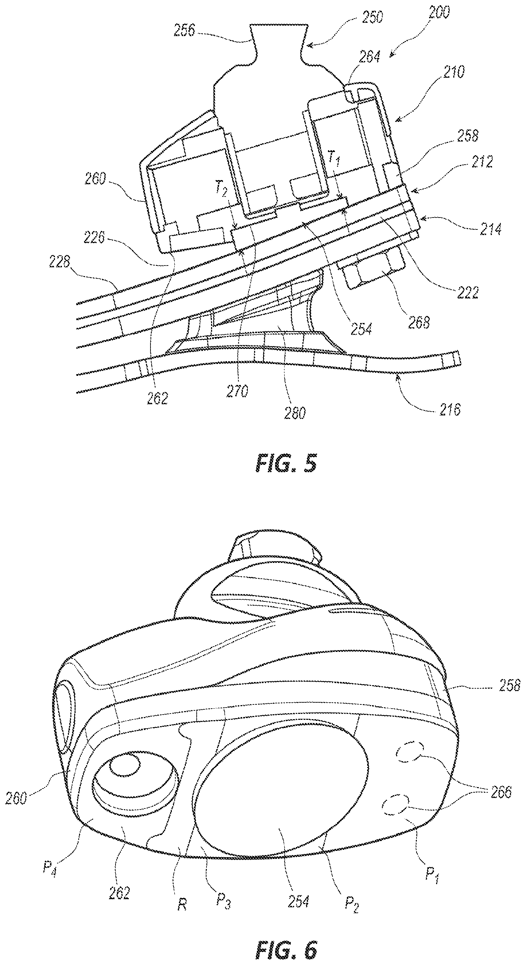

The cavity 270 may have a shape that substantially matches the shape of the damping member 254. For example, the cavity 270 may have a circular disk shape that matches the circular disk shape of the damping member 254 show in FIG. 6. The cavity 270 may have a size (e.g., volume) that is less than the size (e.g., volume) of the damping member 254 before or during application of a force that would compress the damping member 254. As such, at least a portion of the damping member 254 may remain interfacing with and spacing apart the adapter 210 and the top spring member 212 during use of the prosthetic device 200. In other arrangements, the cavity 270 is sized such that the damping member 254, when compressed, can be retained within the cavity 270 such that the contact surface 262 can achieve full contact with the top surface 228 of the top spring member 212 during use.

The damping member 254 may also have a variable thickness across its width. FIG. 5 shows the damping member 254 having a first thickness T₁ at its anterior end and a second thickness T₂ at its posterior end. Many other shapes are possible for the cavity 270 and damping member 254 including, for example, rectangular, polygonal, triangular, oval or other perimeter shapes, sizes that match or are different, and thicknesses that are variable or constant.

The damping member 254 may include a compressible material. In one example, the damping member 254 includes a polymeric material that is compressible and/or deformable upon application of a force. Some examples materials that are possible for the damping member 254 include the following types of rubbers or elastomers: polyisoprene, polybutadiene, polychloroprene, butyl, chloro butyl, bromo butyl, styrene-butadiene, nitrile, hydrogenated nitrile, ethylene propylene, ethylene propylene diene, epichlorohydrin, polyacrylic, polysulfide, silicone, thermoplastic and thermoset urethane, fluorosilicone, fluoroelastomers, perfluoroelastomers, polyether block amides, polyester-polyether copolymers, polyamide-polyether copolymers, chlorosulfonated polyethylene, ethylene-vinyl acetate, "thermoplastic elastomers", and polyolefinic elastomers and mixtures or block copolymers of the above. At least some of these materials, once fully constrained from expansion within a cavity, become rigid.

The contact surface 262 may include multiple portions or sections between the posterior end 258 and the anterior end 260. FIG. 6 illustrates a first portion P₁ at the posterior end 258, which is intended to remain in contact with the top surface 228 of the top spring member 212 once the adapter 210 is connected to the top spring member 212 with the fasteners 268. The first portion P₁ may have a planar shape, or a shape that matches the shape of the top surface of the top spring member 212 when the foot is in a rest state. The first portion P₁ transitions to a second portion P₂ in the area of the damping member 254. The second portion P₂ may have a planar shape or may be contoured (i.e., have a radius of curvature). The second portion P₂ may transition to a to a fourth portion P₄ via a third portion P₃. The third portion P₃ may have a radius R. The fourth portion P₄ may be planar or may be contoured.

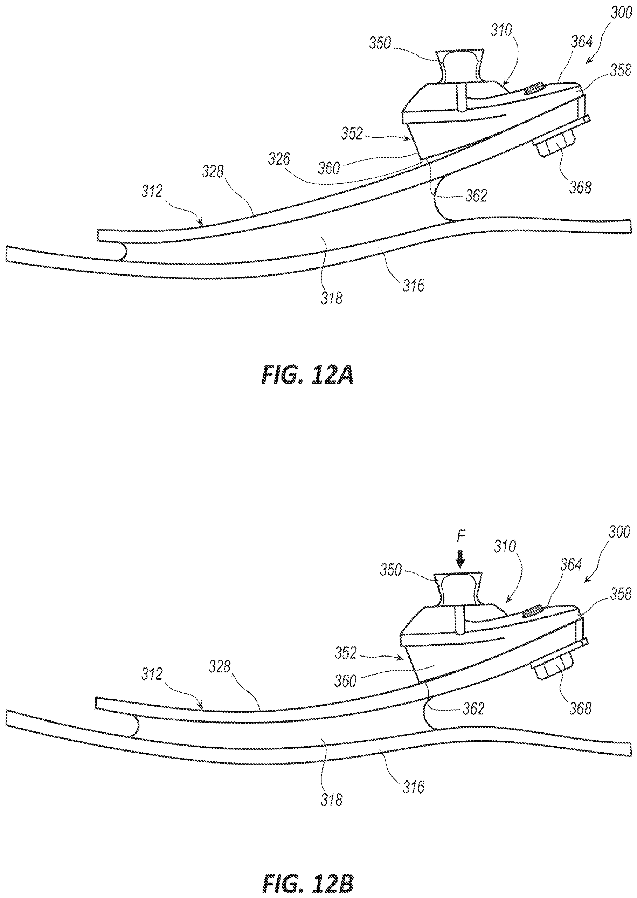

Other configurations are possible for the contact surface 262 as discussed below with reference to FIGS. 12-24.

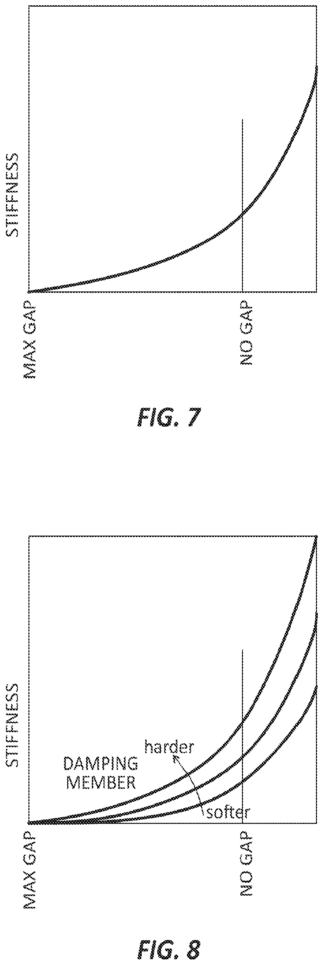

FIG. 7 is a graph that shows how the stiffness of the prosthetic device 100 changes as the user progresses through the gait cycle. Initially, such as during the swing phase and/or during initial heel strike, the gap between the second end of the base and the top surface of the top spring member is at its maximum. As the user moves through the standing phase of the gait cycle, applying more and more force to the prosthetic device, the gap under the adapter's second end (i.e., space 126) is reduced. This portion of the cycle may be referred to as the soft portion of the cycle as it relates to stiffness of the prosthetic foot. The damping member is being compressed or deformed into the cavity of the base during this portion of the cycle. During this phase of the cycle, the stiffness increases at a relatively moderate rate. When the adapter makes full contact with the top spring member (as indicated by the line labeled "no gap" on the graph shown in FIG. 7), the stiffness is then governed solely by the stiffness of the spring members. At this point the stiffness increases at a higher rate.

The properties represented by the graph shown in FIG. 7 may be relevant during low activity or during the mid-stance phase of the gait cycle, to achieve improved and/or optimum roll-over, for the foot to be relatively "soft." As the activity level of the user increases, or as the forces on the distal surface of the prosthetic device move forward towards a toe region during the terminal stance phase, the foot typically needs to become stiffer to achieve optimum stability, provide energy return, and provide adequate support to allow the user to achieve optimum gait. This desired stiffness is provided as the gap (i.e., space 126) reaches zero.

FIG. 8 is a graph showing versatility and adjustability characteristics in accordance with the present disclosure. The initial stiffness of the foot may be changed by simply interchanging the damping member 154 with a harder or softer material. If, for example, the user is an active individual, a harder durometer material may be used for the damping member, thereby optimizing performance of the prosthetic device for that individual. In another example, when the user has a lower activity rate or body weight, a lower durometer material may be used for the damping member, thus providing a softer foot at a performance level that is optimized for that individual.

As noted in FIGS. 7 and 8, once the base 152 is in full contact with the top spring member, the stiffness rate is solely a function of the stiffness of the top spring member (alone or in combination with the other spring members used in the prosthetic device), and the stiffness increases at a higher rate.

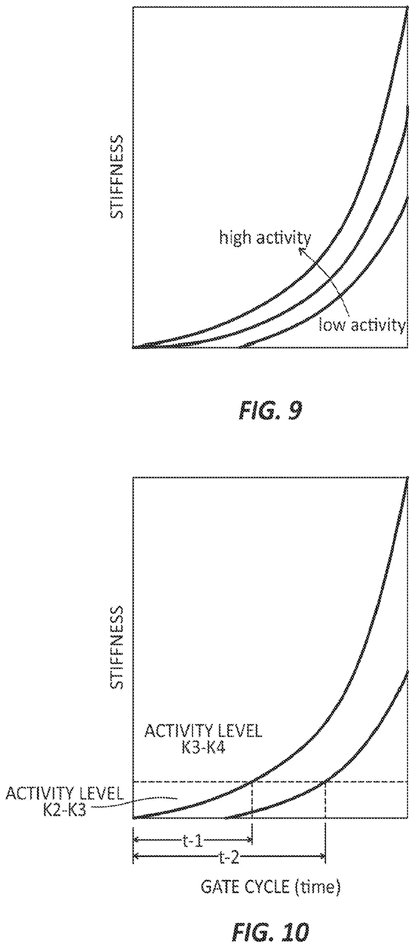

FIG. 9 is a graph showing how the activity level of the user is related to the stiffness of the prosthetic device. The characteristics may be relevant because, for example, during low force activities (e.g., leisurely walking around the house or other non-demand/easy activities) it may be desirable to maximize user comfort. This traditionally has been accomplished by changing the entire prosthetic device to a hydraulic or vertical shock-style foot. These types of prosthetic devices typically consume energy. To achieve optimum performance for demanding or high activities, it is desirable to have a stiffer foot, or transition from soft to stiff relatively quickly without having to change the entire prosthetic device. The prosthetic device as disclosed herein may provide for a softer initial phase in the gait cycle, and then transition to a stiffer, higher performance prosthetic device as the demand is increased. Not only do the prosthetic devices and related adapters disclosed herein generally control the stiffness throughout the gait cycle, but they also allow for simple adjustments (*e.g*., interchanging the damping member to provide different stiffness curve, and characteristics).

FIG. 10 is a graph showing additional versatility and adjustability characteristics of the present disclosure. The initial stiffness is governed by the damping properties of the damping member (*e.g*., durometer of a compressible polymeric member). As the hardness of the damping member is increased, the foot transitions to its stiffer phase sooner. As the stiffness of the damping member is decreased, the foot transitions to its stiffer phase more slowly.

FIG. 10 also shows that small adjustments in the stiffness of the damping member may have significant effect on the performance of the foot as far as responsiveness and the amount of time required during application of force between relatively soft and relatively stiff portions of the stiffness curve.

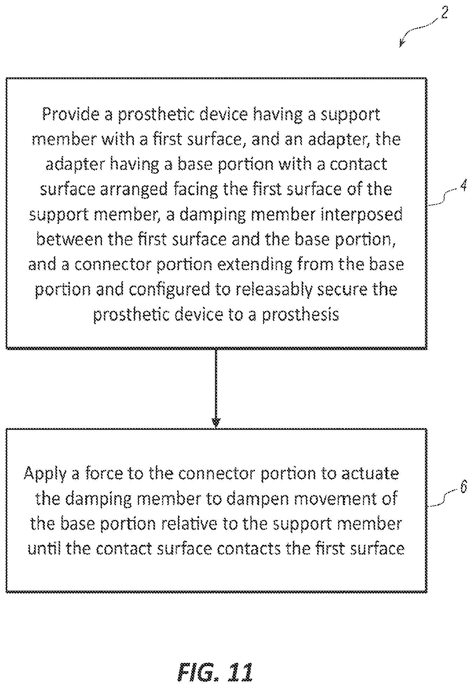

FIG. 11 is a flow diagram illustrating an example method 2 of damping movement and/or changing stiffness characteristics in a prosthetic foot. The method 2 includes, at block 4, providing a prosthetic device having a support member with a first surface and an adapter. The adapter has a base portion with a contact surface arranged facing the first surface of the support member. At least one damping member is interposed between the first surface and the base portion. A connector portion extends from the base portion and is configured to releasably secure the prosthetic device to a prosthesis. At block 6, the method includes applying a force to the connector portion to actuate the damping member thereby controlling movement of the base portion relative to the support portion until the contact surface fully contacts the first surface.

The method may also include, for example, compressing the damping member as part of the actuating. The base member may include a cavity, and actuating the damping member may include filling the cavity in whole or in part with the damping member. The contact surface may have a first end and a second end, the first end may be fixed to the first surface, and the second end may be moveable relative to the first surface in response to applying the force to the connector portion. Actuating the damping member may include changing a shape of the damping member. In one example, the prosthetic device is a prosthetic foot, the support member is an elongate top spring member, the connectors are arranged vertically above the base portion, and the first end of the adapter is positioned posterior of the second end.

Referring now to FIGS. 12A-B, another example prosthetic device 300 is shown. The prosthetic device 300 includes an adapter 310, a top spring number 312, a footplate spring number 316 and a first bond connection 318 between the top and footplate spring numbers 312, 316. The adapter 310 is secured to the top spring member 312 with fasteners 368 along a top surface 328 of the top spring member 312. When the prosthetic device 300 is in an unloaded position as shown in FIG. 12A, a contact surface 362 of the adapter 310 at an anterior end 360 is spaced apart from the top spring number 312. Upon application of a force F as shown in FIG. 12B, the contact surface 362 moves into at least partial contact or full contact with the top surface 328 of the top spring number 312.

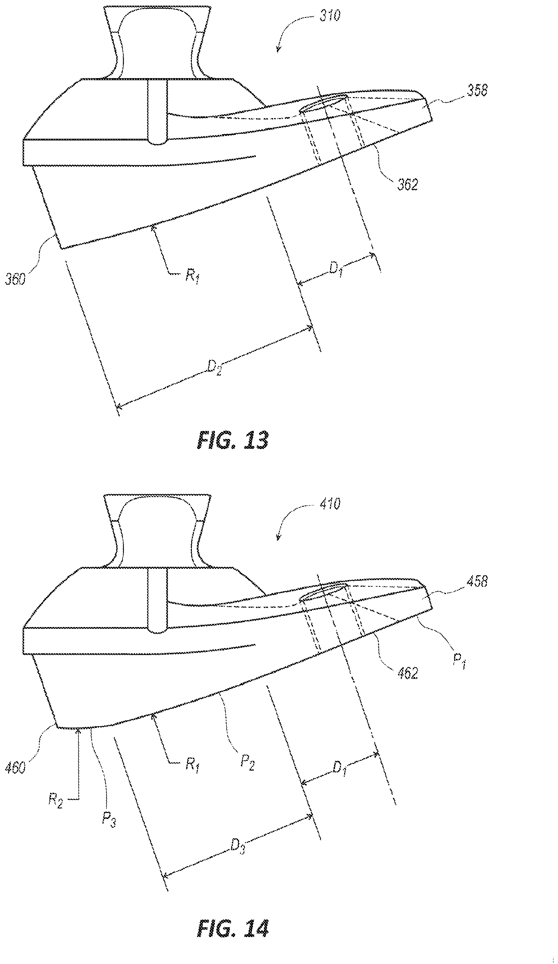

The adapter 310 includes a connector 350 and a base 352. The base includes first and second ends 358, 360 (also referred to as posterior and anterior ends 358, 360), the contact surface 362, and a top surface 364 to which the connector 350 is mounted. The contact surface 362 may have a variety of shapes that influence roll over and stress concentrations. FIG. 13 illustrates the adapter 310 shown in FIGS. 12A and 12B in further detail. The contact surface 362 is planar along the posterior end and to a Distance D₁ from the center of the fasteners 368 in a forward or distal direction to a primary tangent point. At this tangent point, the contact surface 362 transitions from being a flat surface to a contoured surface having a radius of Curvature R₁. The contoured portion of the contact surface 362 has a D₂ from the tangent point to the anterior end 360. The radius of curvature R₁ is typically in the range of about 0.25 cm (1 inch) to about 30.48 cm (12 inches), and more particularly about 25.4 cm (10 inches). The adapters may have fillets at the extreme anterior end where the distal contact surface 362 meets anterior surface 360. The fillet may result from common machining practices to avoid burrs and sharp edges. Fillets with a radius less than approximately 2.54 cm (1 inch) typically have no significant effect on roll over behavior of the foot. A radius smaller than about 2.54 cm (1 inch) on the anterior end of an adapter can reduce localized contact stresses on a spring or support member in comparison with a sharp corner, however further improvements are possible.

Providing the radius of curvature along the contact surface 362 may help optimize the bending stresses in the top spring number 312, which may contribute to maximizing the strength and durability of the prosthetic device 300. Typically, the bending stresses in the top spring number 312 are dependent at least in part on the distance between the reaction forces and a load distribution on the top spring number 312. Increasing the distance between the reaction forces may result in decreased stress and increases in the effective stiffness of the top spring number 312. This load distribution may be determined by the shape of the gap between the curved portion of the contact surface 362 and the top surface 328 of the top spring number 312 (i.e., before application of a force F as shown in FIG. 12B).

FIG. 14 illustrates another example adapter 410 that includes posterior and anterior ends 458, 460 and a contact surface 462. The contact surface 462 includes three distinct portions P₁, P₂, P₃. The first portion P₁ is planar and extends anteriorly to a first tangent point at a distance D₁ from the fasteners. A second portion P₂ extends from the first tangent point to a second tangent point that is spaced in the anterior direction a distance D₃ to a second tangent point. The second portion P₂ has a radius of curvature R₁. A third portion P₃ extends from the second tangent point to the anterior end 460 and has a radius of curvature R₂. The radius of curvature R₂ is different from the radius of curvature R₁, and is typically smaller than R₁. The value of R₁ typically is in the range of about 15.24 cm (6 inches) to about 35.56 cm (14 inches). The value of R₂ is typically in the range of about 10.16 cm (4 inches) to about 25.4 cm (10 inches), and more particularly 17.78 cm (7 inches).

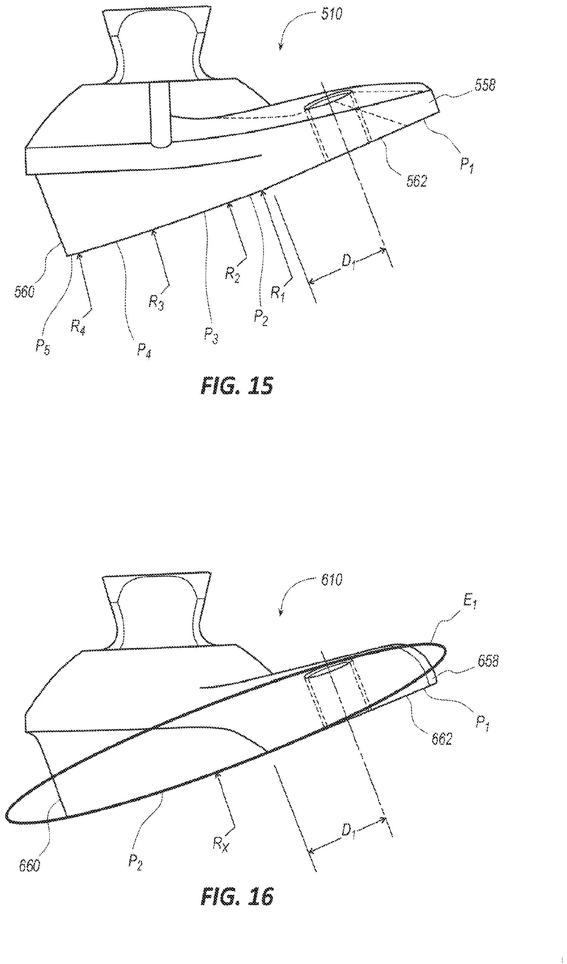

FIG. 15 illustrates another example adapter 510 that includes posterior and anterior ends 558, 560 and a contact surface 562. The contact surface 562 includes five different portions P₁-P₅ that each have a different radius of curvature. The first portion P₁ is typically planar and extends in an anterior direction from the posterior end 558 a distance D₁ to a first tangent point. The second portion P₂ has a first radius R₁. The third of portion P₃ has a radius R₂. A fourth portion has a radius R₃. The fifth portion P₅ has a radius R₄. The radius R₁ typically is in the range of about 2.54 cm (1 inch) to about 10.16 cm (4 inches).

The radius R₂ typically is in the range of about 35.56 cm (14 inches) to about 127 cm (50 inches) and may be a straight line resulting in a planar surface. The radius R₃ typically is in the range of about 15.24 cm (6 inches) to about 25.4 cm (10 inches). And the radius R₄ typically is in the range of about 10.16 cm (4 inches) to about 20.32 cm (8 inches).

The adapter 510 is exemplary of the variety of configurations possible for the contact surface 562 of an adapter in accordance with the present disclosure. The contact surface 562 may have a multi-radius construction. The radius of curvature moving from the first tangent point in an anterior direction may decrease in size, or may be a combination of different radiuses of curvature whether increasing or decreasing, or some combination thereof. At least some of the portions may include a planar construction with an infinite radius of curvature. Such a planar portion may be positioned on one or more sides of a portion of the contact surface having a smaller radius of curvature.

FIG. 16 illustrates another example adapter 610 having posterior and anterior ends 658, 660 and a contact surface 662. A posterior portion P₁ of the contact surface 662 may have a planar construction up to a tangent point at a distance D₁ from the fasteners. The remaining portion P2 of the contact surface 662 may have a continuously variable radius of curvature Rₓ. The radius of curvature Rₓ may be defined by a portion of an elliptical shape E₁. Various elliptical shapes may be used to help define the portion P₂ and the variable radius Rₓ. The ellipse may have a semi-major axis of about 45 mm (1.77 inch) and a semi-minor axis of about 7 mm (0.28 inch).

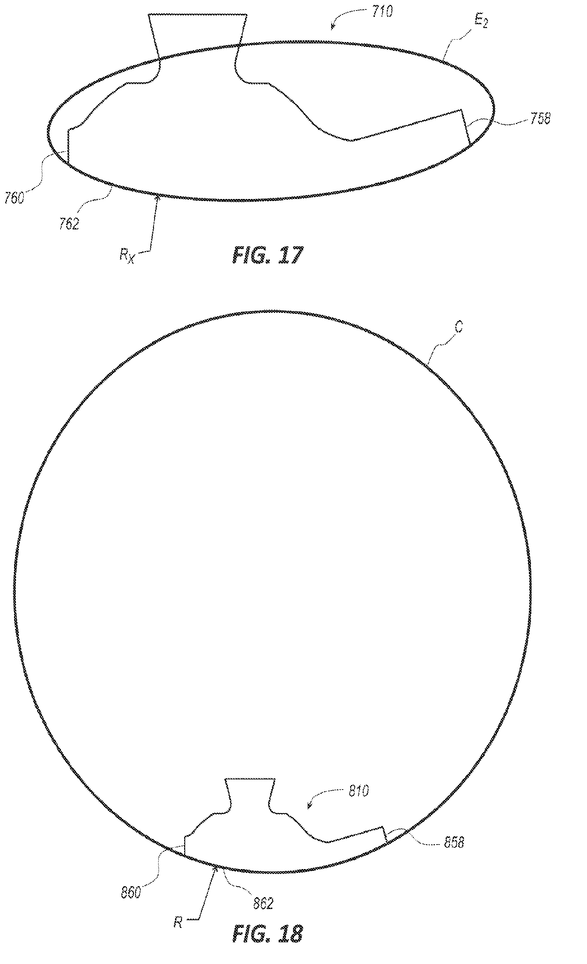

FIG. 17 illustrates another example adapter 710 having posterior and anterior ends 758, 760 and a contact surface 762. The contact surface is defined as a continuously variable radius Rₓ along the entire length of the contact surface 762 from the posterior end 758 to the anterior end 760. The adapter 710 may have no portion of the contact surface 762 that is planar, or at least only a small portion that is planar (e.g., in the area where the fastener secures the adapter to a top spring member). The radius of curvature Rₓ may be defined by an elliptical shape E₂ that is different from that shown in FIG. 16. Other elliptical shapes having different radiuses of curvature may be used to define the contact surface 762, or portions thereof. In at least some examples, multiple elliptical shapes may be used to define various portions of the contact surface 762.

FIG. 18 illustrates another example adapter 810 having posterior and anterior ends 858, 860 and a contact surface 862. The contact surface 862 has a radius of curvature R. The radius curvature R may be defined by a circular shape C. The circular shape C has a constant radius of curvature. The radius of curvature R may extend along the entire length of the contact surface 862 from the posterior end 858 to the anterior end 860. In other arrangements, only a portion of the contact surface 862 is defined by the radius of curvature C while other portions are defined with a different radius of curvature or are planar (e.g., in the area of the fastener). In one example, the radius of curvature R is in the range of about 15.24 cm (6 inches) to about 35.56 cm (14 inches).

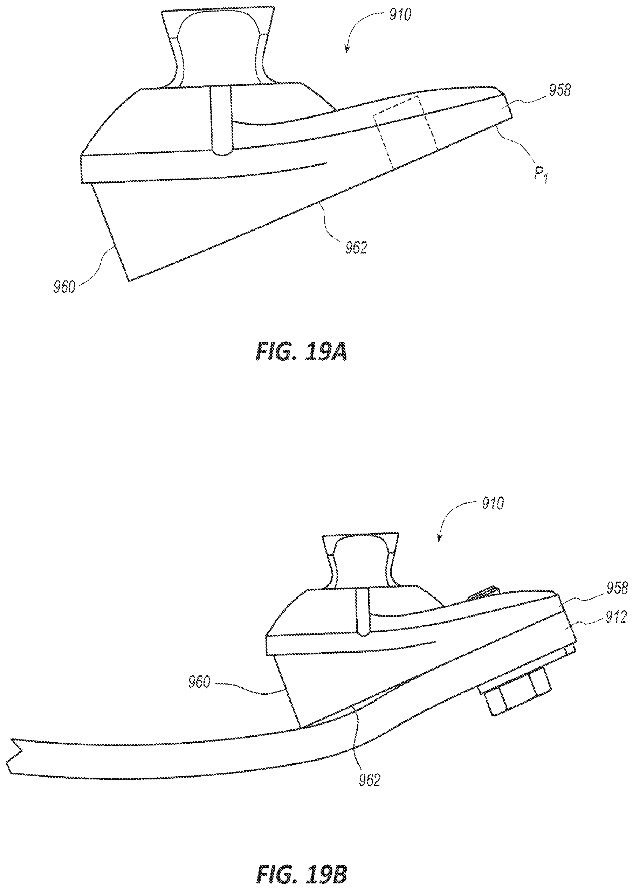

FIG. 19A illustrates another example adapter 910 having posterior and anterior ends 958, 960 and a contact surface 962. The contact surface 962 is planar along its entire length from the posterior end 958 to the anterior end 960. The planar shape of the contact surface 962 provides constant contact between the contact surface 962 and the top surface of a top spring member to which the adapter 910 is mounted. In some embodiments in which a damping member is interposed between the adapter contact surface 962 and the top surface of the top spring member, application of a force to the adapter may adjust a spacing between the contact surface 962 and the top surface of the top spring member. The use of a planar contact surface 962 may result in stress concentrations, poor roll over characteristics for the prosthetic foot, and other disadvantages as disclosed herein.

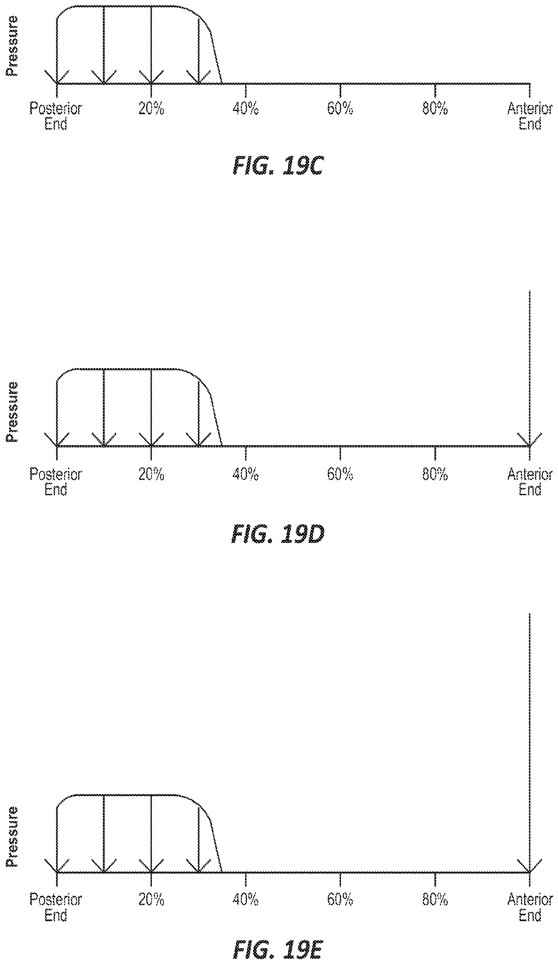

FIG. 19B illustrates adapter 910 connected to spring member 912 when a force is applied to the prosthetic device. FIGS. 19C-E show the progression of pressure distribution between the contact surface of the adapter and the top spring member as a load is applied to the prosthetic device. The graphs shown in FIGS. 19C-E are exemplary only, but may have particular relevance to the embodiment shown in FIGS. 19A-B. The load may be referred to as the force F that is applied to the adapter as shown in FIG. 12B.

FIG. 19C depicts the pressure distribution with no load on the prosthetic device. The horizontal axis of the graph shows the percentage of the distal adapter length. The pressure shown in 19C is the result of preloaded fasteners attaching the adapter to the spring member and it is assumed the fastener preload pressure distribution does not change as the load and moment on the spring member increases. FIG. 19D demonstrates the pressure distribution with a moderate load on the device. FIG. 19E shows the pressure distribution with a high load applied to the device. This planar contact surface of the adapter results in a large point load at the anterior end of the adapter resulting in a high stress concentration in the spring member at the anterior contact point 966.

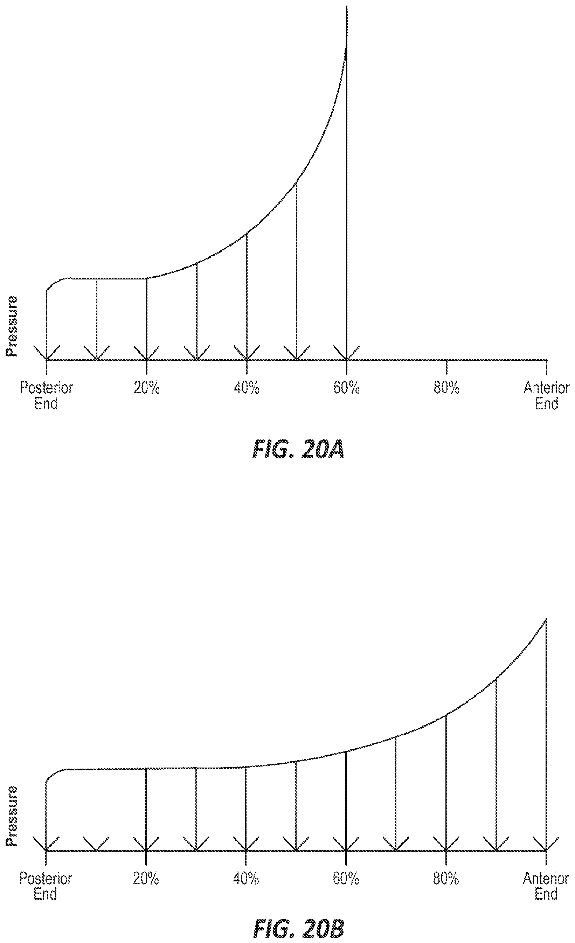

One approach to reducing the stress concentration at the anterior end of an adapter is to incorporate a radius on the anterior distal end of an adapter, which may result in a gap between the spring member and the anterior end of the adapter. FIGS. 20A and 20B show pressure distributions when the adapter has a single radius on the anterior end which extends forward of a planar section. FIG. 20A also shows the pressure distribution when a high load is applied to a prosthetic foot when the radius is insufficient (i.e., too small) for the moment applied to the spring member. The spring member typically does not roll up onto the radius to any significant degree, and only the planar section of the distal surface of the adapter is in contact with the spring member. Due to the short length of the planar section, the reaction forces are relatively high, although in comparison to FIG. 19E the stress concentration is alleviated slightly by a tangent transition between the planar section and the radius.

FIG. 20B illustrates the pressure distribution when a single radius on the anterior distal surface of the adapter is excessively large relative to the moment applied to the spring member. The spring member rolls up onto the radius and the reaction forces move to the anterior end of the adapter. Because the radius is larger than optimum, the pressure distribution, and hence the bending stresses in the spring member, are high at the anterior end of the adapter. This is similar to the loading to FIG. 19B, although less extreme. A single radius on the anterior end of the adapter may be optimized for a single magnitude of load or moment, where alternate designs may be more optimum when a variety of loading conditions are considered. The gap between the adapter and the spring member may be further optimized to improve strength and flexibility for walking, running, or carrying a heavy load, or alternatively for other athletic activities or specific activities, or some combination of many possible loading conditions.

An alternative to a single radius at the anterior portion of the contact surface of the adapter and a potential improvement provided by the present disclosure is a curved contact surface with two radii as shown in, for example, FIG. 14. In some examples, the radii are each equal to or greater than about 0.64 cm (0.25 inch), and more particularly equal to or grater than about 2.54 cm (1 inch).

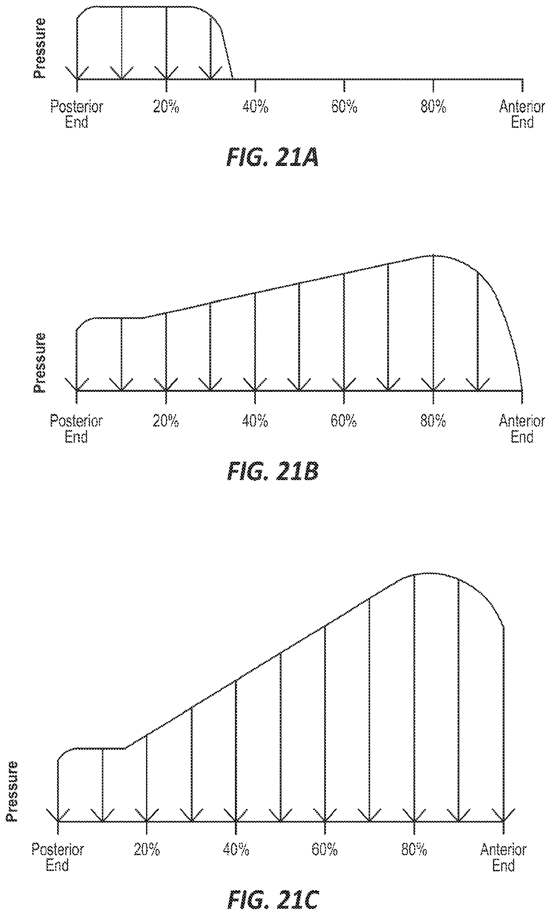

The graphs of FIGS. 21A-C show the progression of the pressure distribution between curved portions of the contact surface of the adapter and the top foot spring as the load on the toe of the foot and the moment on the top spring member increases from mid-stance to terminal stance. FIG. 21A shows the pressure distribution when the foot is unloaded. There may be no contact between the anterior section of adapter and the spring member, and the pressure distribution may be the result of the preload of the fasteners attaching the adapter to the spring member. In this example, the two radii are tangentially connected at a distance D₃ as shown in FIG. 14, which is anterior to the tangent point of the first radius. The most anterior radius portion (P₃) is smaller than the more posterior radius portion P₂. FIG. 21B shows the pressure distribution when the upper spring member is subjected to a medium moment, for example, during the transition between mid-stance and terminal stance. The pressure at the anterior end of the adapter contact surface is reduced as compared to FIG 20A due to the smaller radius portion P₃.

FIG. 21C shows the pressure distribution when the upper spring member is subjected to high moment during terminal stance. Again, the smaller radius portion P3 may reduce the stress concentration at the end of the adapter in comparison to an adapter with either a single radius (i.e., compared with FIG. 20B) or an adapter with a planar contact surface along the entire length, e.g., adapter 910 shown in FIGS. 19A-E. Moreover, an adapter with two radii at the anterior end of the distal surface may provide improved pressure distribution, flexibility, and/or reduced bending stress in a spring member when subjected to a variety of loading conditions, and hence an improved balance of high cycle fatigue durability for typical walking loads, low cycle fatigue durability for athletic activities, and/or high static failure strength may be achieved.

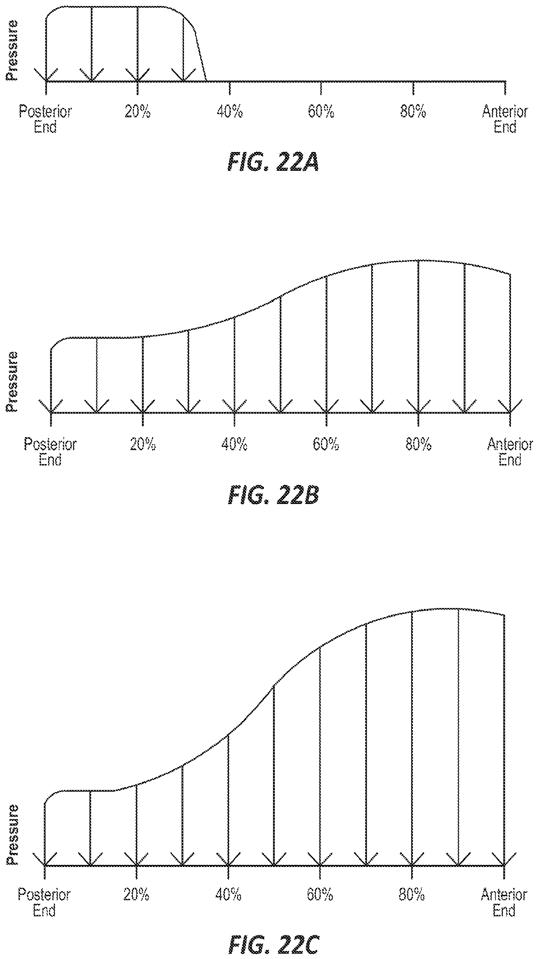

FIGS. 22A-C illustrate pressure distribution when the adapter has a continuously variable radius of curvature along the contact surface, such as a conic section and more specifically with the elliptical design shown in FIG. 16. FIGS. 22A-C show the progression of the pressure distribution between the curved portion of the adapter contact surface and the top spring member as moment on the upper spring member increases from mid stance to terminal stance when the adapter contact surface has an elliptical curvature at the anterior end. FIG. 22A is the pressure distribution when the foot is unloaded showing only the preload from the adapter attachment fasteners. FIG. 22B shows the pressure distribution when the foot has a medium moment. FIG. 22C is the pressure distribution when the upper spring member is subjected to a high moment. Because an ellipse has continuously variable radius the pressure distribution is smooth without abrupt changes. The pressure decreases slightly from the maximum at the anterior end of the adapter, which reduces the stress concentration at the anterior end of the adapter and provides a desirable combination of flexibility and minimizing stress in the spring member. In one example the ellipse may have a semi-major axis of about 45 mm (1.77 inch) and a semi-minor axis of about 7 mm (0.28 inch).

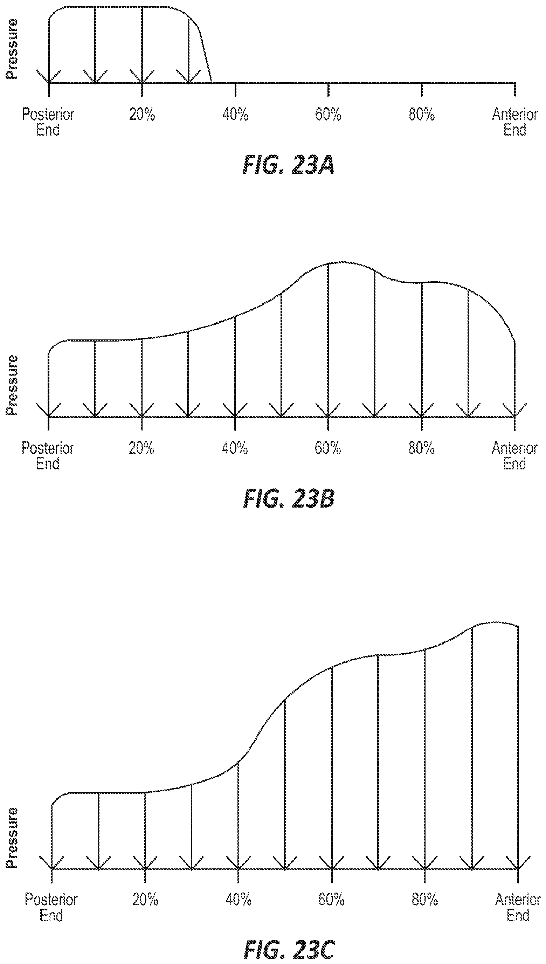

Another alternative to a single radius or planar contact surface of the anterior portion of the adapter is a contact surface with multiple radii such as the embodiments shown in FIG. 15. In this example, there are four radii tangentially connected to each other as depicted in FIG. 15, and the size of each radius is different to allow both optimum pressure distribution and flexibility in the top spring member. FIGS. 23A-C show the progression of the pressure distribution between the curved portion of the adapter contact surface and the top spring member as the moment on the top spring member increases. FIG. 23A shows the pressure distribution when the foot is unloaded and the pressure distribution is the result of fastener preload. FIG. 23B shows the pressure distribution when the spring member experiences a medium bending moment. FIG. 23C shows the pressure distribution when the spring member has a large bending moment. FIG. 23C shows high pressure distribution near, but not at, the anterior end of the adapter. This design maximizes flexibility of the top spring member during mid-stance until the top spring member makes contact with radius R₃ during progression from mid-stance to terminal stance. Once the top spring member makes contact with R₃ and the moment on the top spring member increases further as the user progresses through the gait cycle, this surface begins to react to a significant amount of the moment applied to the top spring member. Because high pressure occurs at a significant distance from the fasteners used to secure the adapter to the top spring member, the resultant adapter reaction forces have advantageous leverage to react the moment. Smaller radius R₄ at the anterior end of the adapter results in pressure at the anterior end of the adapter, which is less than the peak pressure, which avoids a stress concentration at the anterior end of the adapter.

The shape of the gap between the adapter contact surface and the upper surface of the top spring member determines at least in part the flexibility of the top spring member and the stresses in the spring member that contacts the adapter. As the top spring member rolls into contact with the adapter contact surface, the lever arm acting on the top spring member is shortened, thus stiffening the top spring member. The bending moment and bending stresses that the top spring member experience are also reduced. Therefore, the foot may be more flexible in mid-stance using the adapter contact surface configurations disclosed herein, particularly when there is little moment applied to the toe of the prosthetic foot, thus assisting the user during mid-stance roll over. The prosthetic foot then stiffens as the moment increases and the lever arm shortens, thus resulting in increased support during terminal stance. The maximum bending moment and stresses may occur during terminal stance. Therefore, it may be helpful to minimize these stresses while maximizing flexibility during mid-stance when the user has little leverage over the prosthetic foot. Designing the gap between the adapter and the spring member that contacts the adapter offers opportunities to optimize both bending stresses and flexibility to maximize prosthetic foot performance.

The foregoing embodiments demonstrate the effect of modifying the distal contact surface shape of a prosthetic adapter when the spring member in contact with the adapter is flat. The same effect can be achieved by defining the shape of the upper surface of a spring member in contact with an adapter having a flat distal surface, or by defining the contact surfaces of both the adapter and the spring member. Defining the shape of a gap between the contact surfaces of a prosthetic adapter and a spring member which contacts the adapter allows a designer to optimize the strength and flexibility of a prosthetic foot.

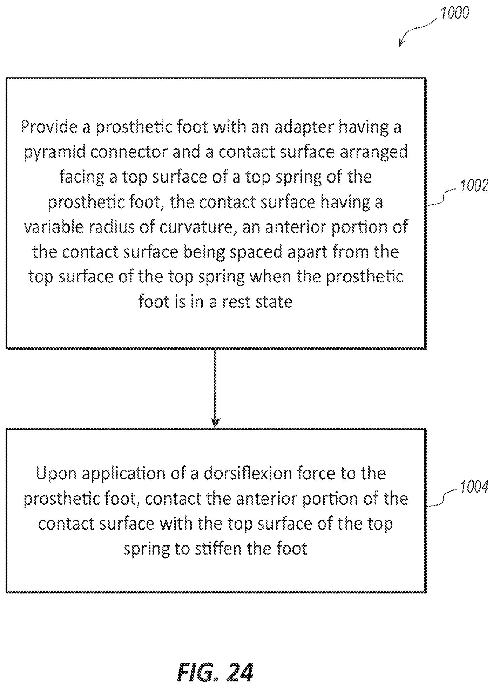

FIG. 24 shows steps of an example method 1000 for operating a prosthetic device in accordance with the present disclosure. The method 1000 includes, at block 1002, providing the prosthetic foot with an adapter having a pyramid connector and a contact surface arranged facing a top surface of a top spring of the prosthetic foot. The contact surface has a variable radius of curvature. An anterior portion of the contact surface is spaced apart from the top surface of the top spring when the prosthetic foot is in a rest state. At block 1004, the method includes, upon application of a dorsal flection force to the prosthetic foot, contact in the anterior portion of the contact surface with the top surface of the top spring to stiffen the foot.

The method 1000 may also include providing the prosthetic foot with a resilient member interposed between the top spring and the adapter, wherein the resilient member provides dampening between the adapter and the top spring during operation of the prosthetic foot. The variable radius surface may include a recess, and the resilient member may extend into the recess.

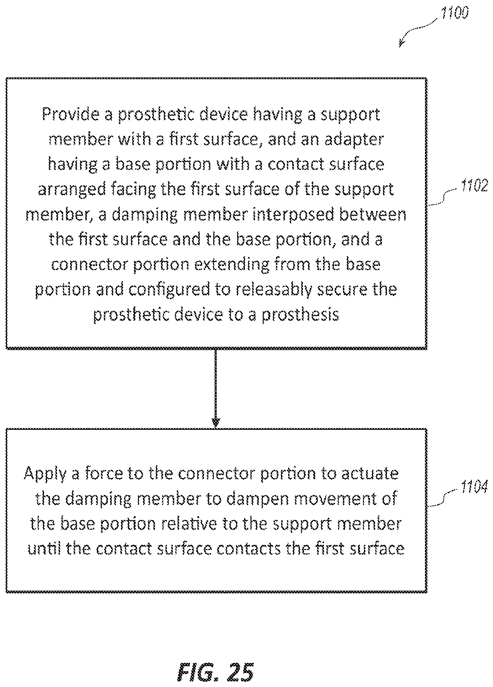

FIG. 25 shows steps of another example method 1100 of dampening movement in a prosthetic foot in accordance with the present disclosure. The method 1100 may include, at block 1102, providing a prosthetic foot having a support member having a first surface, and an adapter having a base portion with a contact surface arranged facing the first surface of the support member, a damping member interposed between the first surface and the base portion, and a connector portion extending from the base portion and configured to releasably secure the prosthetic device to a prosthesis. At block 1104, the method includes applying a force to the connector portion to actuate the damping member to dampen movement of the base portion relative to the support member until the contact surface contacts the first surface.

The method 1100 may also provide for actuating the damping member by compressing the damping member. The base member may include a cavity in actuating the damping member may include filling the cavity with the damping member. The contact surface may have a first end, a second end, and a contoured shape between the first and second ends, and the first and is fixed relative to the first surface, and the second end is moveable relative to the first surface in response to applying a force to the connector portion. The connector portion may include deforming the support member until the first surface has a contoured shape that matches a contoured shape of the contact surface. The prosthetic device may be a prosthetic foot, and the support member may be an elongate spring member, and the connector may be arranged vertically above the base portion with the first end of the adapter positioned posterior of the second end.

The foregoing description, for purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the present systems and methods and their practical applications, to thereby enable others skilled in the art to best utilize the present systems and methods and various embodiments with various modifications as may be suited to the particular use contemplated.

Unless otherwise noted, the terms "a" or "an," as used in the specification and claims, are to be construed as meaning "at least one of." In addition, for ease of use, the words "including" and "having," as used in the specification and claims, are interchangeable with and have the same meaning as the word "comprising." In addition, the term "based on" as used in the specification and the claims is to be construed as meaning "based at least upon."

## Claims

1. A prosthetic foot, comprising: an elongate support member of carbon filament material having a top surface (128, 228); an adapter (110, 210) comprising:
a compressible member (154, 254); a base portion;
a cavity (170, 270) sized to receive the compressible member (154, 254);
a connector portion extending from the base portion and configured to releasably secure the prosthetic foot to a prosthesis,
wherein the base portion comprises a contact surface (162, 262) arranged to contact the top surface (128, 228) of the support member,
wherein the volume of the cavity (170, 270) provides a gap (G) between the compressible member (154, 254) and the surfaces of the cavity (170, 270) prior to use, the compressible member (154, 254) is deformable to fill the cavity (170, 270) when compressed and wherein the application of a force to the connector portion compresses the compressible member (154, 254) to dampen movement of the base portion relative to the support member until the contact surface (162, 262) contacts the top surface (128, 228).

2. The prosthetic foot of claim 1, **characterized in that** the base portion has a first end and a second end, the first end being fixed relative to the support member, and the second end being movable relative to the support member.

3. The prosthetic foot of claim 1, **characterized in that** the compressible member (154, 254) having a volume filling the cavity (170, 270) completely when compressed.

4. The prosthetic foot of claim 1, **characterized in that** the contact surface (162, 262) has a curved shape.

5. The prosthetic foot of claim 1, **characterized in that** the compressible (154, 254) is sized that, when compressed, is retained entirely within the cavity (170, 270).

6. The prosthetic foot of claim 1, **characterized in that** the compressible member (154, 254) comprises a polymeric material.

7. The prosthetic foot of claim 1, **characterized in that** the cavity (170, 270) has a hemispherical shape.

8. The prosthetic foot of claim 2, **characterized in that** the contact surface (162, 262) is spaced away from the top surface (128, 228) at the second end when the prosthetic foot is in a rest state prior to application of the force.

## Patentansprüche

1. Prothesenfuß mit einem länglichen Stützelement aus Carbonfasermaterial mit einer oberen Oberfläche (128, 228); einem Adapter (110, 210), umfassend:
ein komprimierbares Element (154, 254); ein Basisteil;
einen Hohlraum (170, 270), der so bemessen ist, dass er das komprimierbare Element (154, 254) aufnehmen kann;
einen Verbindungsabschnitt, der sich von dem Basisteil erstreckt und so gestaltet ist, dass er den Prothesenfuß lösbar an einer Prothese befestigt,
wobei das Basisteil eine Kontaktfläche (162, 262) umfasst, die so angeordnet ist, dass sie die obere Fläche (128, 228) des Stützelements berührt, wobei das Volumen des Hohlraums (170, 270) vor der Verwendung einen Spalt (G) zwischen dem komprimierbaren Element (154, 254) und den Oberflächen des Hohlraums (170, 270) bereitstellt, wobei das komprimierbare Element (154, 254) verformbar ist, um den Hohlraum (170, 270) zu füllen, wenn es zusammengedrückt wird, und
wobei das Aufbringen einer Kraft auf den Verbindungsabschnitt das komprimierbare Element (154, 254) zusammendrückt, um die Bewegung des Basisteils relativ zu dem Stützelement zu dämpfen, bis die Kontaktfläche (162, 262) die obere Oberfläche (128, 228) berührt.

2. Prothesenfuß nach Anspruch 1, **dadurch gekennzeichnet, dass** das Basisteil ein erstes Ende und ein zweites Ende aufweist, wobei das erste Ende relativ zu dem Stützelement fixiert ist und das zweite Ende relativ zu dem Stützelement beweglich ist.

3. Prothesenfuß nach Anspruch 1, **dadurch gekennzeichnet, dass** das komprimierbare Element (154, 254) ein Volumen aufweist, das den Hohlraum (170, 270) vollständig ausfüllt, wenn es zusammengedrückt ist.

4. Prothesenfuß nach Anspruch 1, **dadurch gekennzeichnet, dass** die Kontaktfläche (162, 262) eine gekrümmte Form aufweist.

5. Prothesenfuß nach Anspruch 1, **dadurch gekennzeichnet, dass** das komprimierbare Element (154, 254) so bemessen ist, dass es, wenn es zusammengedrückt ist, vollständig in dem Hohlraum (170, 270) gehalten wird.

6. Prothesenfuß nach Anspruch 1, **dadurch gekennzeichnet, dass** das komprimierbare Element (154, 254) ein polymeres Material aufweist.

7. Prothesenfuß nach Anspruch 1, **dadurch gekennzeichnet, dass** der Hohlraum (170, 270) eine Halbkugelform aufweist.

8. Prothesenfuß nach Anspruch 2, **dadurch gekennzeichnet, dass** die Kontaktfläche (162, 262) von der oberen Oberfläche (128, 228) am zweiten Ende beabstandet ist, wenn sich der Prothesenfuß vor dem Aufbringen der Kraft in einem Ruhezustand befindet.

## Revendications

1. Pied prothétique, comprenant un élément de support allongé en matériau de filament de carbone ayant une surface supérieure (128, 228) ; un adaptateur (110, 210) comprenant :
un élément compressible (154, 254) ; une partie de base ;
une cavité (170, 270) dimensionnée pour recevoir l'élément compressible (154, 254) ;
une partie de connexion s'étendant à partir de la partie de base et configurée pour fixer de manière amovible le pied prothétique à une prothèse,
la partie de base présentant une surface de contact (162, 262) agencée pour entrer en contact avec la surface supérieure (128, 228) de l'élément de support ;
dans lequel
le volume de la cavité (170, 270) offre un espace (G) entre l'élément compressible (154, 254) et les surfaces de la cavité (170, 270) avant utilisation,
l'élément compressible (154, 254) est déformable pour remplir la cavité (170, 270) lorsqu'il est comprimé, et
l'application d'une force à la partie de connexion fait que l'élément compressible (154, 254) est comprimé pour amortir le mouvement de la partie base par rapport à l'élément de support jusqu'à ce que la surface de contact (162, 262) entre en contact avec la surface supérieure (128, 228).

2. Pied prothétique selon la revendication 1,
**caractérisé en ce que** la partie de base présente une première extrémité et une deuxième extrémité, la première extrémité étant fixe par rapport à l'élément de support, et la deuxième extrémité étant mobile par rapport à l'élément de support.

3. Pied prothétique selon la revendication 1,
**caractérisé en ce que** l'élément compressible (154, 254) présente un volume qui remplit complètement la cavité (170, 270) lorsqu'il est comprimé.

4. Pied prothétique selon la revendication 1,
**caractérisé en ce que** la surface de contact (162, 262) présente une forme incurvée.

5. Pied prothétique selon la revendication 1,
**caractérisé en ce que** l'élément compressible (154, 254) est dimensionné de telle sorte que, lorsqu'il est comprimé, il est entièrement retenu à l'intérieur de la cavité (170, 270).

6. Pied prothétique selon la revendication 1,
**caractérisé en ce que** l'élément compressible (154, 254) comprend un matériau polymère.

7. Pied prothétique selon la revendication 1,
**caractérisé en ce que** la cavité (170, 270) présente une forme hémisphérique.

8. Pied prothétique selon la revendication 2,
**caractérisé en ce que** la surface de contact (162, 262) est espacée de la surface supérieure (128, 228) à la deuxième extrémité lorsque le pied prothétique est à l'état de repos avant l'application de la force.
